(19) **Europäisches Patentamt**

**European Patent Office**

**Office européen des brevets**

(11) **EP 1 141 256 B1**

(12) **EUROPEAN PATENT SPECIFICATION**

(45) Date of publication and mention
of the grant of the patent:
**18.10.2006 Bulletin 2006/42**

(51) Int Cl.:
***C12N 9/54*** (2006.01)

(86) International application number:
**PCT/DK1999/000711**

(21) Application number: **99960941.5**

(22) Date of filing: **20.12.1999**

(87) International publication number:
**WO 2000/037621 (29.06.2000 Gazette 2000/26)**

(54) **SUBTILASE ENZYMES OF THE I-S1 AND I-S2 SUB-GROUPS HAVING AN ADDITIONAL AMINO ACID RESIDUE IN AN ACTIVE SITE LOOP REGION**

SUBTILASE ENZYME DER I-S1 und IS2 UNTERGRUPPEN, MIT EINEM ZUSÄTZLICHEN AMINOSAÜREREST IN EINER AKTIVEN SCHLEIFENREGION

ENZYMES SUBTILASES DES SOUS GROUPES I-S1 ET I-S2 POSSEDANT UN RESIDU ACIDE AMINE SUPPLEMENTAIRE DANS UN REGION EN BOUCLE DE SITE ACTIF

(84) Designated Contracting States:
**AT BE CH CY DE DK ES FI FR GB GR IE IT LI LU MC NL PT SE**

(30) Priority: **18.12.1998 DK 167799**
**20.05.1999 DK 70699**

(43) Date of publication of application:
**10.10.2001 Bulletin 2001/41**

(73) Proprietor: **Novozymes A/S**
**2880 Bagsvaerd (DK)**

(72) Inventors:
• **ANDERSEN VILBOUR, Kim**
**DK-2700 Bronshoj (DK)**

• **MIKKELSEN, Frank**
**2500 Valby (DK)**
• **HANSEN KAMP, Peter**
**2880 Bagsvaerd (DK)**
• **ANDERSEN, Carsten**
**2880 Bagsvaerd (DK)**
• **NORREGAARD-MADSEN, Mads**
**DK-3460 Birkerod (DK)**

(56) References cited:
**EP-A1- 0 405 901    WO-A1-98/20115**
**WO-A1-98/55634    WO-A1-99/27082**
**WO-A2-95/30011    WO-A2-96/28566**

SEQUENCE LISTING

<110> Novo Nordisk A/S

<120> Subtilase enzymes of the I-S1 and I-S2 sub-groups having an additional amino acid residue in an active site loop region.

<130> 5692.204

<140>
<141>

<160> 2

<170> PatentIn Ver. 2.1

<210> 1
<211> 275
<212> PRT
<213> Bacillus licheniformis

<400> 1

```
Ala Gln Thr Val Pro Tyr Gly Ile Pro Leu Ile Lys Ala Asp Lys Val
 1               5                  10                  15

Gln Ala Gln Gly Phe Lys Gly Ala Asn Val Lys Val Ala Val Leu Asp
            20                  25                  30

Thr Gly Ile Gln Ala Ser His Pro Asp Leu Asn Val Val Gly Gly Ala
        35                  40                  45

Ser Phe Val Ala Gly Glu Ala Tyr Asn Thr Asp Gly Asn Gly His Gly
        50                  55                  60

Thr His Val Ala Gly Thr Val Ala Ala Leu Asp Asn Thr Thr Gly Val
65                  70                  75                  80

Leu Gly Val Ala Pro Ser Val Ser Leu Tyr Ala Val Lys Val Leu Asn
                85                  90                  95

Ser Xaa Ser Gly Ser Gly Thr Tyr Ser Gly Ile Val Ser Gly Ile Glu
            100                 105                 110

Trp Ala Thr Thr Asn Gly Met Asp Val Ile Asn Met Ser Leu Gly Gly
            115                 120                 125

Pro Ser Gly Ser Thr Ala Met Lys Gln Ala Val Asp Asn Ala Tyr Ala
```

```
                  130                    135                       140

        Arg Gly Val Val Val Val Ala Ala Ala Gly Asn Ser Gly Ser Ser Gly
        145                 150                 155                 160

        Asn Thr Asn Thr Ile Gly Tyr Pro Ala Lys Tyr Asp Ser Val Ile Ala
                        165                 170                 175

        Val Gly Ala Val Asp Ser Asn Ser Asn Arg Ala Ser Phe Ser Ser Val
                        180                 185                 190

        Gly Ala Glu Leu Glu Val Met Ala Pro Gly Ala Gly Val Tyr Ser Thr
                        195                 200                 205

        Tyr Pro Thr Ser Thr Tyr Ala Thr Leu Asn Gly Thr Ser Met Ala Ser
                210                 215                 220

        Pro His Val Ala Gly Ala Ala Ala Leu Ile Leu Ser Lys His Pro Asn
        225                 230                 235                 240

        Leu Ser Ala Ser Gln Val Arg Asn Arg Leu Ser Ser Thr Ala Thr Tyr
                        245                 250                 255

        Leu Gly Ser Ser Phe Tyr Tyr Gly Lys Gly Leu Ile Asn Val Glu Ala
                        260                 265                 270

        Ala Ala Gln
                275


<210> 2
<211> 270
<212> PRT
<213> Bacillus lentus

<400> 2


        Ala Gln Ser Val Pro Trp Gly Ile Ser Arg Val Gln Ala Pro Ala Ala
        1               5                   10                  15

        His Asn Arg Gly Leu Thr Gly Ser Gly Val Lys Val Ala Val Leu Asp
                        20                  25                  30

        Thr Gly Ile Ser Thr His Pro Asp Leu Asn Ile Arg Gly Gly Ala Ser
                        35                  40                  45

        Phe Val Pro Gly Glu Pro Ser Thr Gln Asp Gly Asn Gly His Gly Thr
                50                  55                  60
```

```
His Val Ala Gly Thr Ile Ala Ala Leu Asn Asn Ser Ile Gly Val Leu
65              70          75          80

Gly Val Ala Pro Ser Ala Glu Leu Tyr Ala Val Lys Val Leu Gly Ala
            85          90          95

Xaa Ser Gly Ser Gly Ser Val Ser Ser Ile Ala Gln Gly Leu Glu Trp
        100         105         110

Ala Gly Asn Asn Gly Met His Val Ala Asn Leu Ser Leu Gly Ser Pro
        115         120         125

Ser Pro Ser Ala Thr Leu Glu Gln Ala Val Asn Ser Ala Thr Ser Arg
    130         135         140

Gly Val Leu Val Val Ala Ala Ser Gly Asn Ser Gly Ala Gly Ser Ile
145         150         155         160

Ser Tyr Pro Ala Arg Tyr Ala Asn Ala Met Ala Val Gly Ala Thr Asp
            165         170         175

Gln Asn Asn Asn Arg Ala Ser Phe Ser Gln Tyr Gly Ala Gly Leu Asp
        180         185         190

Ile Val Ala Pro Gly Val Asn Val Gln Ser Thr Tyr Pro Gly Ser Thr
    195         200         205

Tyr Ala Ser Leu Asn Gly Thr Ser Met Ala Thr Pro His Val Ala Gly
    210         215         220

Ala Ala Ala Leu Val Lys Gln Lys Asn Pro Ser Trp Ser Asn Val Gln
225         230         235         240

Ile Arg Asn His Leu Lys Asn Thr Ala Thr Ser Leu Gly Ser Thr Asn
            245         250         255

Leu Tyr Gly Ser Gly Leu Val Asn Ala Glu Ala Ala Thr Arg
            260         265         270
```

**Description**

<u>TECHNICAL FIELD</u>

This invention relates to novel subtilase enzymes of the I-S1 and I-S2 sub-groups having at least one additional amino acid residue in position 98 of the active site loop (b) region from position 95 to 103. These proteases are useful exhibiting excellent or improved wash performance when used in detergents; cleaning and detergent compositions. The invention further relates to genes coding for the expression of said enzymes when inserted into a suitable host cell or nor-human organism; and such host cells transformed therewith and capable of expressing said enzyme variants, and methods for producing the novel enzymes.

<u>BACKGROUND OF THE INVENTION</u>

In the detergent industry enzymes have for more than 30 years been implemented in washing formulations. Enzymes used in such formulations comprise proteases, lipases, amylases, cellulases, as well as other enzymes, or mixtures thereof. Commercially most important enzymes are proteases.

An increasing number of commercially used proteases are protein engineered variants of naturally occurring wild type proteases, e.g. DURAZYM® (Novo Nordisk A/S), RELASE® (Novo Nordisk A/S), MAXAPEM® (Gist-Brocades N.V.), PURAFECT® (Genencor International, Inc.).

Further a number of protease variants are described in the art, such as in EP 130756 (GENENTECH) (corresponding to US Reissue Patent No. 34,606 (GENENCOR)); EP 214435 (HENKEL); WO 87/04461 (AMGEN); WO 87/05050 (GENEX); EP 260105 (GENENCOR); Thomas, Russell, and Fersht (1985) Nature 318 375-376; Thomas, Russell, and Fersht (1967) *J. Mol. Biol.* 193 803-813; Russel and Fersht *Nature* 328 496-500 (1987); WO 88/08028 (Genex); WO 88/08033 (Amgen); WO 95/27049 (SOLVAY S.A.); WO 95/30011 (PROCTER & GAMBLE COMPANY) ; WO 95/30010 (PROCTER & GAMBLE 5 COMPANY); WO 95/29979 (PROCTER & GAMBLE COMPANY); US 5.543.302 (SOLVAY S.A.); EP 251 446 (GENENCOR); WO 89/06279 (NOVO NORDISK A/S); EP 525 610 A1 (SOLVAY); and WO 94/02618 (GIST-BROCADES N.V.). Also, WO 91/00345 (NOVO NORDISK A/S) discloses mutated subtilisins in which the net electrostatic charge has been changed by modifying the amino acid residues of the enzyme in at least one of a number of indicated positions.

However, even though a number of useful protease variants have been described, there is still a need for new improved proteases or protease variants for a number of industrial uses.

Therefore, an object of the present invention, is to provide improved proteases or protein engineered protease variants, especially for use in the detergent industry.

<u>SUMMARY OF THE INVENTION</u>

The present inventors have found that subtilisins wherein at least one of the active site loops are longer than those presently known, exhibit improved wash performance properties in detergent compositions. The identification thereof was done in constructing subtilisin variants, especially of the subtilisin 309 (BLSAVI or Savinase®), exhibiting improved wash performance properties in detergent compositions relative to the parent wild type enzyme. This has been described in our earlier application DK133Z/97.

It has now been found that certain subtilases or variants thereof of the I-S1 (true "subtilisins") and I-S2 (high alkaline subtilisins) sub-groups having at least one additional amino acid residue in position 9B (or rather between positions 98 and 99) of the active site loop (b) region from position 95 to 103, exhibit surprisingly improved wash performance in comparison to those presently known and those described in said application.

The improved proteases according to the invention may be obtained by isolation from natural resources or by the introduction of at least one further amino acid residue (an insertion) in the active site loop (b) between positions 98 and 99 in a wild type subtilase (for a definition of the active site loops and the numbering of positions see below).

Although this finding was done in subtilisin 309 it is predicted that it will be possible to produce or isolate similar advantageous subtilases or subtilase variants.

Furthermore it will be possible to specifically screen natural isolates to identify novel wild type subtilases comprising an active site loop (b) which is longer than the corresponding active site loop in known wild type subtilases, such as subtilisin 309, which subtilases can be considered to have an inserted amino acid residue between positions 98 and 99, and exhibiting excellent wash performance in a detergent, in comparison to their closest related known subtilisin, such as subtilisin 309.

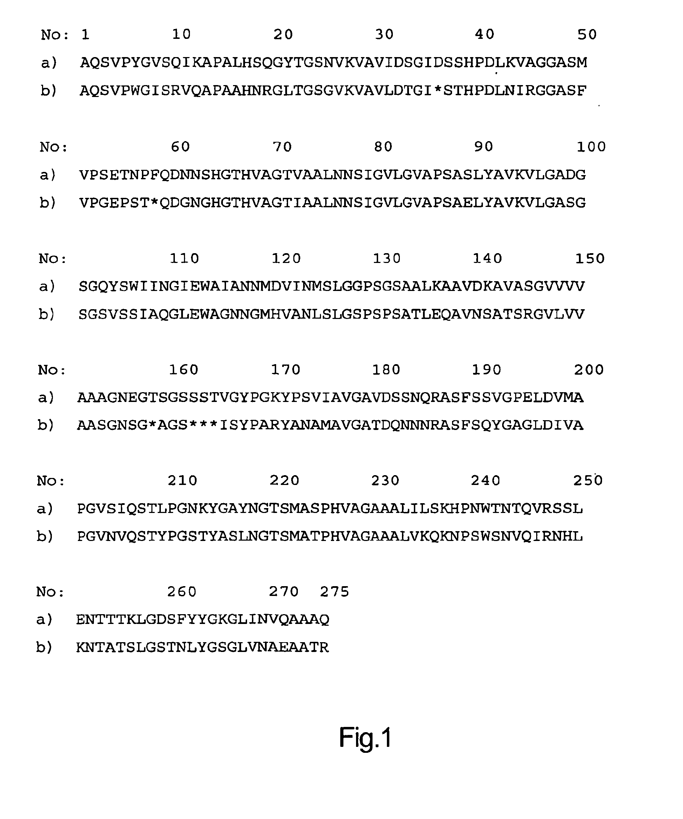

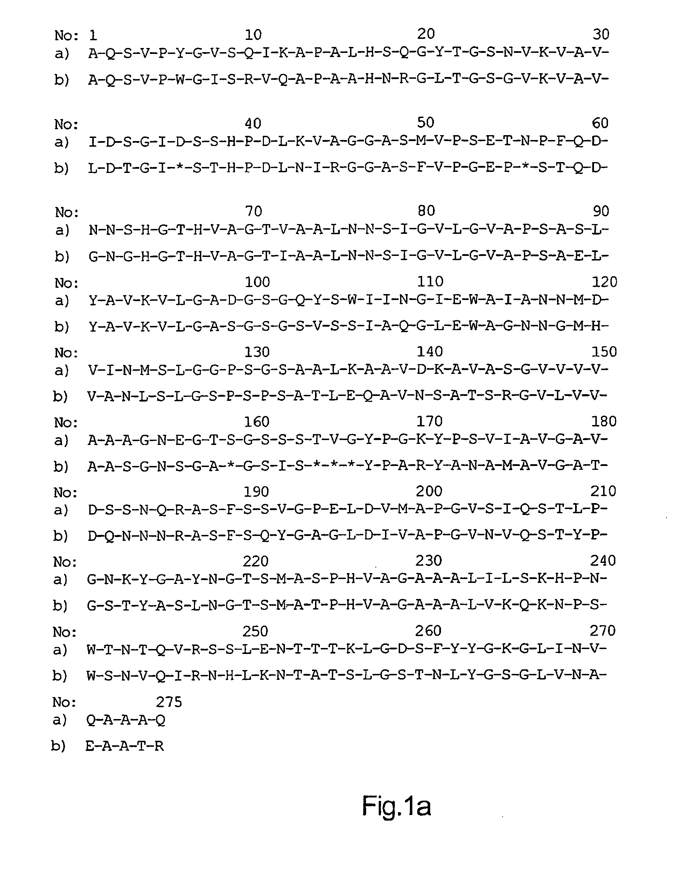

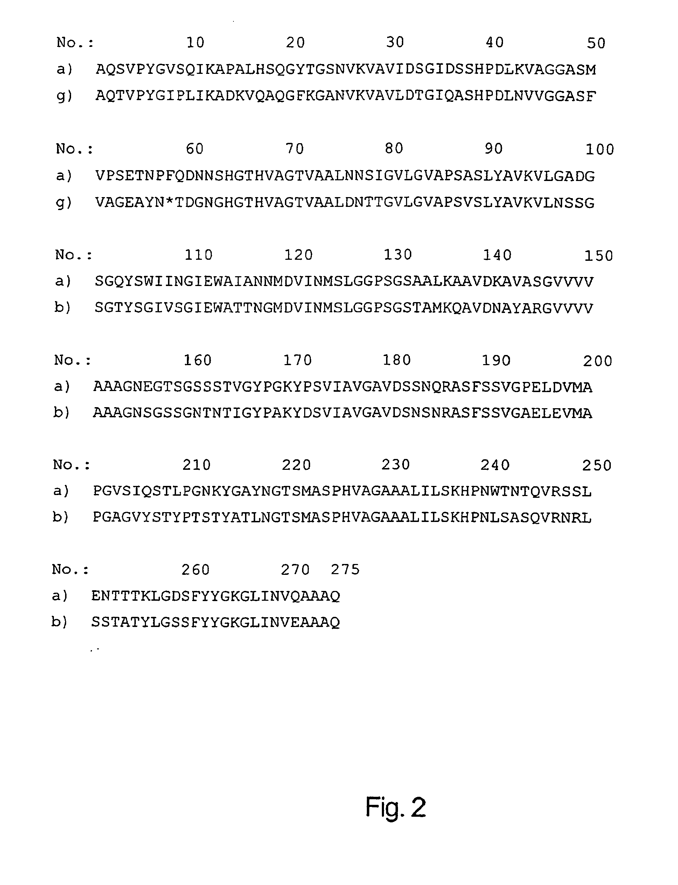

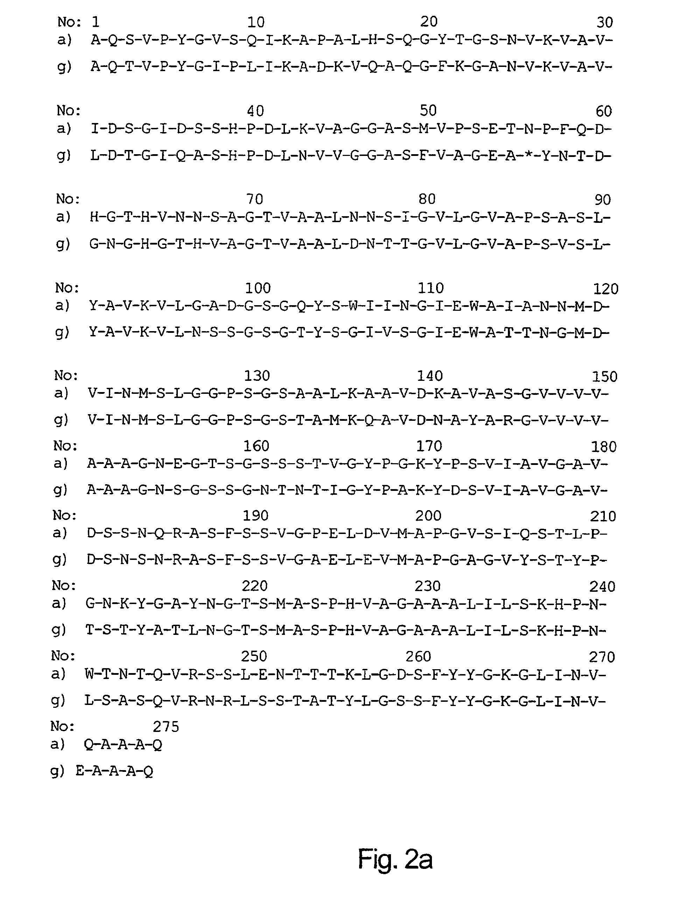

Concerning alignment and numbering reference is made to Figs. 1, 1a, 2 and 2a below showing alignments between subtilisin BPN' (BASBPN)(a) and subtilisin 309 (BLSAVI)(b), and alignments between subtilisin BPN'(a) (BASBPN) and subtilisin Carlsberg (g). In Figs. 1 and 2 the alignments were established by the use of the GAP routine of the GCG package as indicated below, whereas the alignments of Figs. 1a and 2a are the same as shown in WO 91/00345. These alignments are in this patent application used as a reference for numbering the residues.

The seven active site loops (a) to (g) (including both the end amino acid residues indicated) are here defined to encompass the amino acid residues in the segments given below

(a) the region between amino acid residue 33 and 43;
(b) the region between amino acid residue 95 and 103;
(c) the region between amino acid residue 125 and 132;
(d) the region between amino acid residue 153 and 173;
(e) the region between amino acid residue 181 and 195;
(f) the region between amino acid residue 202 and 204;
(g) the region between amino acid residue 218 and 219.

Accordingly, in a first aspect the invention relates to an isolated (*i.e.* greater than 10 % pure) subtilase enzymes of the I-S1 and I-S2 sub-groups having at least one additional amino acid residue in position 98 of the active site loop (b) region from position 95 to 103, whereby said additional amino acid residue(s) correspond to the insertion of at least one amino acid residue between positions 98 and 99.

In a second aspect the invention relates to an isolated DNA sequence encoding a subtilase variant of the invention.

In a third aspect the invention relates to an expression vector comprising an isolated DNA sequence encoding a subtilase variant of the invention.

In a fourth aspect the invention relates to a microbial host cell transformed with an expression vector according to the fourth aspect.

In a further aspect the invention relates to the production of the subtilisin enzymes of the invention.

The enzymes of the invention can generally be produced by either cultivation of a microbial strain from which the enzyme was isolated and recovering the enzyme in substantially pure form; or by inserting an expression vector according to the fourth aspect of the invention into a suitable microbial host, cultivating the host to express the desired subtilase enzyme, and recovering the enzyme product.

Further the invention relates to a composition comprising a subtilase or subtilase variant of the invention.

Even further the invention relates to the use of the enzymes of the invention for a number of industrial relevant uses, in particular for use in cleaning compositions and cleaning compositions comprising the mutant enzymes, especially detergent compositions comprising the mutant subtilisin enzymes.

## DEFINITONS

Prior to discussing this invention in further detail, the following terms and conventions will first be defined.

## NOMENCLATURE OF AMINO ACIDS

| | | |
|---|---|---|
| A | = Ala | = Alanine |
| V | = Val | = Valine |
| L | = Leu | = Leucine |
| I | = Ile | = Isoleucine |
| P | = Pro | = Proline |
| F | = Phe | = Phenylalanine |
| W | = Trp | = Tryptophan |
| M | = Met | = Methionine |
| G | = Gly | = Glycine |
| S | = Ser | = Serine |
| T | = Thr | = Threonine |
| C | = Cys | = Cysteine |
| Y | = Tyr | = Tyrosine |
| N | = Asn | = Asparagine |
| Q | = Gln | = Glutamine |

(continued)

| D | = Asp | = Aspartic Acid |
|---|-------|-----------------|
| E | = Glu | = Glutamic Acid |
| K | = Lys | = Lysine |
| R | = Arg | = Arginine |
| H | = His | = Histidine |
| X | = Xaa | = Any amino acid |

NOMENCLATURE OF NUCLEIC ACIDS

| A | = Adenine |
|---|-----------|
| G | = Guanine |
| C | = Cytosine |
| T | = Thymine (only in DNA) |
| U | = Uracil (only in RNA) |

NOMENCLATURE AND CONVENTIONS FOR DESIGNATION OF VARIANTS

In describing the various enzyme variants produced or contemplated according to the invention, the following nomenclatures and conventions have been adapted for ease of reference:

A frame of reference is first defined by aligning the isolated or parent wild type enzyme with subtilisin BPN' (BASBPN).

The alignment can be obtained by the GAP routine of the GCG package version 9.1 to number the variants using the following parameters: gap creation penalty = 8 and gap extension penalty = 8 and all other parameters kept at their default values.

Another method is to use known recognised alignments between subtilases, such as the alignment indicated in WO 91/00345. In most cases the differences will not be of any importance.

Such alignments between subtilisin BPN' (BASBPN) and subtilisin 309 (BLSAVI) and subtilisin Carlsberg (BLSCAR), respectively are indicated in Figs. 1, 1a, 2, and 2a. By this a number of deletions and insertions will be defined in relation to BASBPN. In Fig. 1 subtilisin 309 has 6 deletions in positions 36, 58, 158, 162, 163, and 164 in comparison to BASBPN, whereas in Fig. 1a subtilisin 309 has the same deletions in positions 36, 56, 159, 164, 165, and 166 in comparison to BASBPN. In Fig. 2 subtilisin Carlsberg has one deletion in position 58 in comparison to BASBPN, whereas in Fig. 2a subtilisin Carlsberg has the one deletion in position 56 in comparison to BASBPN. These deletions are in Figs. 1, 1a, 2, and 2a indicated by asterixes (*).

The various modifications performed in a wild type enzyme is indicated in general using three elements as follows:

Original amino acid position substituted amino acid

The notation G195E thus means a substitution of a glycine in position 195 with a glutamic acid.

In the case when the original amino acid residue may be any amino acid residue, a short hand notation may at times be used indicating only the position and substituted amino acid,

Position substituted amino acid

Such a notation is particular relevant in connection with modification(s) in homologous subtilases (*vide infra*).

Similarly when the identity of the substituting amino acid residue(s) is immaterial,

Original amino acid position

When both the original amino acid(s) and substituted amino acid(s) may comprise any amino acid, then only the position is indicated, e.g.: 170.

When the original amino acid(s) and/or substituted amino acid(s) may comprise more than one, but not all amino acid(s), then the selected amino acids are indicated inside brackets { },

Original amino acid position {substituted amino acid$_1$, substituted amino acid$_n$}

For specific variants the specific three or one letter codes are used, including the codes Xaa and X to indicate any amino acid residue.

SUBSTITUTIONS:

The substitution of Glutamic acid for glycine in position 195 is designated as:

Gly195Glu or G195E
or the substitution of any amino acid residue acid for glycine in position 195 is designated as:

Gly195Xaa or G195X
or

Gly195 or G195

The substitution of serine for any amino acid residue in position 170 would thus be designated Xaa170Ser or X170S.
or
170Ser or 170S
Such a notation is particular relevant in connection with modification(s) in homologous subtilases (vide infra). 170Ser is thus meant to comprise e.g. both a Lys170Ser modification in BASBPN and Arg170Ser modification in BLSAVI (cf. Fig. 1).
For a modification where the original amino acid(s) and/or substituted amino acid(s) may comprise more than one, but not all amino acid(s), the substitution of glycine, alanine, serine or threonine for arginine in position 170 would be indicated by
Arg170{Gly,Ala,Ser,Thr} or R170(G,A,S,T)
to indicate the variants
R170G, R170A, R170S, and R170T.

DELETIONS:

A deletion of glycine in position 195 will be indicated by:

Gly195* or G195*

Correspondingly the deletion of more than one amino acid residue, such as the deletion of glycine and leucine in positions 195 and 196 will be designated
Gly195*+Leu196* or G195*+L196*

INSERTIONS:

The insertion of an additional amino acid residue such as *e.g.* a lysine after G195 is :

Gly195GlyLys or G195GK; or

when more than one amino acid residue is inserted, such as e.g. a Lys, Ala and Ser after G195 this is :

Gly195GlyLysAlaSer or G195GKAS

In such cases the inserted amino acid residue(s) are numbered by the addition of lower case letters to the position number of the amino acid residue preceding the inserted amino acid residue(s). In the above example the sequences 194 to 196 would thus be:

```
                        194 195 196

        BLSAVI          A - G - L
```

```
                   194 195 195a 195b 195c 196

        Variant         A - G - K - A - S - L
```

In cases where an amino acid residue identical to the existing amino acid residue is inserted it is clear that a degeneracy in the nomenclature arises. If for example a glycine is inserted after the glycine in the above example this would be indicated by G195GG. The same actual change could just as well be indicated as A194AG for the change from

```
                        194 195 196

        BLSAVI          A - G - L
```

to

```
                    194 195  195a 196
        Variant      A - G - G -  L
                    194 194a 195  196
```

Such instances will be apparent to the skilled person, and the indication G195GG and corresponding indications for this type of insertions are thus meant to comprise such equivalent degenerate indications.

FILLING A GAP:

Where a deletion in an enzyme exists in the reference comparison with the subtilisin BPN' sequence used for the numbering, an insertion in such a position is indicated as:

* 36Asp or *36D

for the insertion of an aspartic acid in position 36

MULTIPLE MODIFICATIONS

Variants comprising multiple modifications are separated by pluses, *e.g.:*

Arg170Tyr+Gly195Glu or R170Y+G195E

representing modifications in positions 170 and 195 substituting tyrosine and glutamic acid for arginine and glycine, respectively.
or *e.g.* Tyr167{Gly,Ala,Ser,Thr}+Arg170{Gly,Ala,Ser,Thr} designates the variants

| | |
|---|---|
| Tyr167Gly+Arg170Gly, | Tyr167Gly+Arg170Ala, |
| Tyr167Gly+Arg170Ser, | Tyr167Gly+Arg170Thr, |
| Tyr167Ala+Arg170Gly, | Tyr167Ala+Arg170Ala, |
| Tyr167Ala+Arg170Ser, | Tyr167Ala+Arg170Thr, |

(continued)

| | |
|---|---|
| Tyr167Ser+Arg170Gly, | Tyr167Ser+Arg170Ala, |
| Tyr167Ser+Arg170Ser, | Tyr167Ser+Arg170Thr, |
| Tyr167Thr+Arg170Gly, | Tyr167Thr+Arg170Ala, |
| Tyr167Thr+Arg170Ser, and | Tyr167Thr+Arg170Thr. |

This nomenclature is particular relevant relating to modifications aimed at substituting, replacing, inserting or deleting amino acid residues having specific common properties, such as residues of positive charge (K, R, H), negative charge (D, E), or conservative amino acid modification(s) of e.g. Tyr167{Gly,Ala,Ser,Thr}+Arg170{Gly,Ala,Ser,Thr}, which signifies substituting a small amino acid for another small amino acid. See section "Detailed description of the invention" for further details.

Proteases

Enzymes cleaving the amide linkages in protein substrates are classified as proteases, or (interchangeably) peptidases (see Walsh, 1979, *Enzymatic Reaction Mechanisms.* W.H. Freeman and Company, San Francisco, Chapter 3).

Numbering of amino acid positions/residues

If nothing else is mentioned the amino acid numbering used herein correspond to that of the subtilase BPN' (BASBPN) sequence. For further description of the BPN' sequence see Figs. 1 and 2, or Siezen *et al., Protein Engng.* **4** (1991) 719-737.

Serine proteases

A serine protease is an enzyme which catalyzes the hydrolysis of peptide bonds, and in which there is an essential serine residue at the active site (White, Handler and Smith, 1973 *"Principles of Biochemistry,"* Fifth Edition, McGraw-Hill Book Company, NY, pp. 271-272).
The bacterial serine proteases have molecular weights in the 20,000 to 45,000 Dalton range. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) *Bacteriological Rev.* **41** 711-753).

Subtilases

A sub-group of the serine proteases tentatively designated subtilases has been proposed by Siezen et al., Protein *Engng.* **4** (1991) 719-737 and Siezen et al. Protein *Science* **6** (1997) 501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen et al. now is a subgroup of the subtilases. A wide variety of subtilases have been identified, and the amino acid sequence of a number of subtilases has been determined. For a more detailed description of such subtilases and their amino acid sequences reference is made to Siezen et al.(1997).
One subgroup of the subtilases, I-S1 or " true# subtilisins, comprises the "classical" subtilisins, such as subtilisin 168 (BSS168), subtilisin BPN', subtilisin Carlsberg (ALCALASE®, NOVO NORDISK A/S), and subtilisin DY (BSSDY).
A further subgroup of the subtilases, I-S2 or high alkaline subtilisins, is recognised by Siezen *et al. (supra).* Sub-group I-S2 proteases are described as highly alkaline subtilisins and comprises enzymes such as subtilisin PB92 (BAALKP) (MAXACAL®, Gist-Brocades NV), subtilisin 309 (SAVINASE®, NOVO NORDISK A/S), subtilisin 147 (BLS147) (ESPERASE®, NOVO NORDISK A/S), and alkaline elastase YaB (BSEYAB).

List of acronyms for subtilases:

I-S1

Subtilisin 168, BSS168 (BSSAS (Subtilisin amylosacchariticus), BSAPRJ (Subtilisin J), BSAPRN (Subtilisin NAT), BMSAMP (Mesentericopeptidase),
Subtilisin BPN', BASBPN,
Subtilisin DY, BSSDY,
Subtilisin Carlsberg, BLSCAR (BLKERA (Keratinase), BLSCA1, BLSCA2, BLSCA3),
BSSPRC, Serine protease C
BSSPRD, Serine protease D

I-S2

Subtilisin Sendai, BSAPRS
Subtilisin ALP 1, BSAPRQ,
Subtilisin 147, Esperase® BLS147 (BSAPRM (SubtilisinAprM), BAH101),
Subtilisin 309, Savinase®, BLS309/BLSAVI (BSKSMK (M-protease), BAALKP(Subtilisin PB92, Bacillus alkalophilic alkaline protease), BLSUBL (Subtilisin BL)),
Alkaline elastase YaB, BYSYAB,

"SAVINASE®"

SAVINASE® is marketed by NOVO NORDISK A/S. It is subtilisin 309 from B. Lentus and differs from BAALKP only in one position (N87S, see Fig. 1 herein). SAVINASE® has the amino acid sequence designated b) in Fig. 1.

Parent subtilase

The term "parent subtilase" describes a subtilase defined according to Siezen et al. (1991 and 1997). For further details see description of "SUBTILASES" immediately above. A parent subtilase may also be a subtilase isolated from a natural source, wherein subsequent modification have been made while retaining the characteristic of a subtilase. Alternatively the term "parent subtilase" may be termed "wild type subtilase".

Modification(s) of a subtilase variant

The term "modification(s)" used herein is defined to include chemical modification of a subtilase as well as genetic manipulation of the DNA encoding a subtilase. The modification(s) can be replacement(s) of the amino acid side chain(s), substitution(s), deletion(s) and/or insertions in or at the amino acid(s) of interest.

Subtilase variant

In the context of this invention, the term subtilase variant or mutated subtilase means a subtilase that has been produced by an organism which is expressing a mutant gene derived from a parent microorganism which possessed an original or parent gene and which produced a corresponding parent enzyme, the parent gene having been mutated in order to produce the mutant gene from which said mutated subtilase protease is produced when expressed in a suitable host.

Homologous subtilase sequences

Specific active site loop regions, and amino acid insertions in said loops of SAVINASE® subtilase are identified for modification herein to obtain a subtilase variant of the invention.
However, the invention is not limited to modifications of this particular subtilase, but extend to other parent (wildtype) subtilases, which have a homologous primary structure to that of SAVINASE®. The homology between two amino acid sequences is in this context described by the parameter "identity " .
In order to determine the degree of identity between two subtilases the GAP routine of the GCG package version 9.1 can be applied (infra) using the same settings. The output from the routine is besides the amino acid alignment the calculation of the "Percent Identity" between the two sequences.
Based on this description it is routine for a person skilled in the art to identify suitable homologous subtilases and corresponding homologous active site loop regions, which can be modified according to the invention.

Wash performance

The ability of an enzyme to catalyze the degradation of various naturally occurring substrates present on the objects to be cleaned during e.g. wash or hard surface cleaning is often referred to as its washing ability, wash-ability, detergency, or wash performance. Throughout this application the term wash performance will be used to encompass this property.

Isolated DNA sequence

The term "isolated", when applied to a DNA sequence molecule, denotes that the DNA sequence has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78, 1985). The term "an isolated DNA sequence" may alternatively be termed "a cloned DNA sequence".

Isolated protein

When applied to a protein, the term "isolated" indicates that the protein has been removed from its native environment.
In a preferred form, the isolated protein is substantially free of other proteins, particularly other homologous proteins (i.e. "homologous impurities" (see below)).
An isolated protein is greater than 10 % pure, preferably greater than 20 % pure, more preferably greater than 30 % pure, as determined by SDS-PAGE. Further it is preferred to provide the protein in a highly purified form, i.e., greater than 40% pure, greater than 60% pure, greater than 80% pure, more preferably greater than 95% pure, and even more preferably greater than 99% pure, as determined by SDS-PAGE.
The term "isolated protein" may alternatively be termed "purified protein".

Homologous impurities

The term "homologous impurities" means any impurity (e.g. another polypeptide than the polypeptide of the invention) which originate from the homologous cell where the polypeptide of the invention is originally obtained from.

Obtained from

The term "obtained from" as used herein in connection with a specific microbial source, means that the polynucleotide and/or polypeptide produced by the specific source, or by a cell in which a gene from the source has been inserted.

Substrate

The term "Substrate" used in connection with a substrate for a protease is should be interpreted in its broadest form as comprising a compound containing at least one peptide bond susceptible to hydrolysis by a subtilisin protease.

Product

The term " product " used in connection with a product derived from a protease enzymatic reaction should in the context of this invention be interpreted to include the products of a hydrolysis reaction involving a subtilase protease. A product may be the substrate in a subsequent hydrolysis reaction.

**BRIEF DESCRIPTION OF THE DRAWING**

Fig. 1 shows an alignment between subtilisin BPN' (a) and Savinase® (b) using the GAP routine mentioned above.
Fig. 1a shows the alignment between subtilisin BPN' and Savinase® as taken from WO 91/00345.

Fig. 2 shows an alignment between subtilisin BPN' and subtilisin Carlsberg using the GAP routine mentioned above.
Fig. 2a shows the alignment between subtilisin BPN' and subtilisin Carlsberg as taken from WO 91/00345.

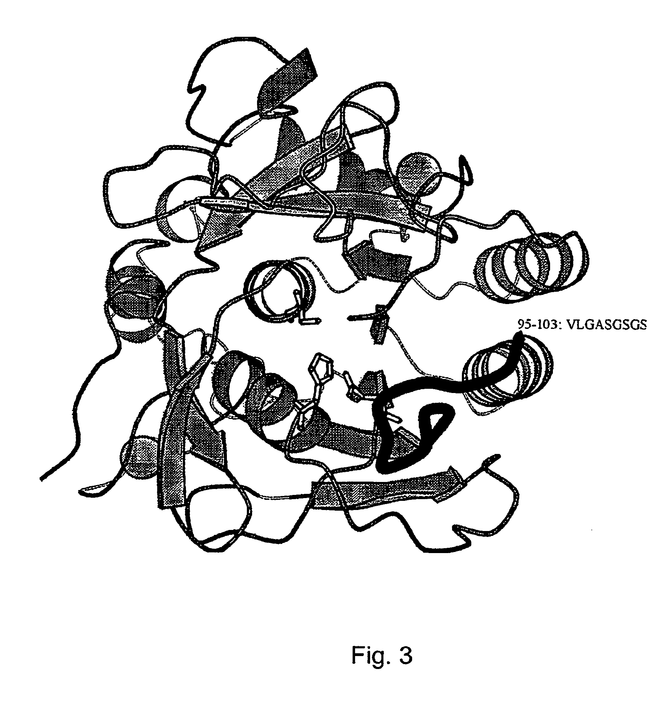

Fig. 3 shows the three dimensional structure of Savinase (Protein data bank (PDB) entry 1SVN). In the Figure the active site loop (b) is indicated.

## DETAILED DESCRIPTION OF THE INVENTION

The subtilases of the invention in a first aspect relates to an isolated (*i.e.* greater than 10 % pure) subtilase enzyme of the I-S1 and I-S2 sub-groups having at least one additional amino acid residue in position 98 of the active site loop (b) region from position 95 to 103, whereby said additional amino acid residue(s) correspond to the insertion of at least one amino acid residue between positions 98 and 99.

In other words the subtilases of the invention are characterized by comprising an active site loop (b) region of more than 9 amino acid residue and wherein the additional amino acid residue is or can be considered as being inserted between positions 98 and 99 as compared to the parent or a known wild type subtilase.

A subtilase of the first aspect of the invention may be a parent or wildtype subtilase identified and isolated from nature.

Such a parent wildtype subtilase may be specifically screened for by standard techniques known in the art.

One preferred way of doing this may be by specifically PCR amplify DNA regions known to encode active site loops in subtilases from numerous different microorganism, preferably different Bacillus strains.

Subtilases are a group of conserved enzymes, in the sense that their DNA and amino acid sequences are homologous. Accordingly it is possible to construct relatively specific primers flanking active site loops.

One way of doing this is by investigating an alignment of different subtilases (see *e.g.* Siezen et al. *Protein Science* **6** (1997) 501-523). It is from this routine work for a person skilled in the art to construct PCR primers flanking the active site loop corresponding to the active site loop (b) between amino acid residue 95 to 103 in any of the group I-S1 or I-S2 groups, such as from BLSAVI. Using such PCR primers to amplify DNA from a number of different microorganism, preferably different Bacillus strains, followed by DNA sequencing of said amplified PCR fragments, it will be possible to identify strains which produce subtilases of these groups comprising a longer, as compared to e.g. BLSAVI, active site region corresponding to the active site loop region from positions 95 to 103, and where an insertion can be considered to exist between positions 98 and 99. Having identified the strain and a partial DNA sequence of such a subtilase of interest, it is routine work for a person skilled in the art to complete cloning, expression and purification of such a subtilase of the invention.

However, it is envisaged that a subtilase enzyme of the invention predominantly is a variant of a parent subtilase.

Accordingly, in one embodiment the invention relates to an isolated subtilase enzyme according to the first aspect of the invention, wherein said subtilase enzyme is a constructed variant having a longer active site loop (b) than its parent enzyme by having at least one amino acid insertion between amino acid residues 98 and 99.

The subtilases of the invention exhibit excellent wash performance in a detergent, and if the enzyme is a constructed variant an improved wash performance in a detergent in comparison to its closest related subtilase, such as subtilisin 309.

Different subtilase products will exhibit a different wash performance in different types of detergent compositions. A subtilase of the invention has improved wash performance, as compared to its closest relative in a majority of such different types of detergent compositions.

Preferably a subtilase enzyme of the invention has improved wash performance, as compared to its closest relative in the detergent composition shown in Example 3 herein (vide infra).

In order to determine if a given subtilase amino acid sequence (irrelevant whether said subtilase sequence is a parent wildtype subtilase sequence or a subtilase variant sequence produced by any other method than by site directed mutagenesis) is within the scope of the invention, the following procedure may be used:

    i) align said subtilase sequence to the amino acid sequence of subtilisin BPN' (see section "Definitions" herein (vide supra);
    ii) based on the alignment performed in step i) identify the active site loop (b), in said subtilase sequence corresponding to the active site loop (b) region of subtilisin BPN' comprising the region (both of the end amino acids included) between amino acid residue from 95 to 103;
    iii) determine if the active site loop (b) in said subtilase sequence, identified in step ii) is longer than the corresponding active site loop in BLSAVI and if said prolongation corresponds to the insertion of at least one amino acid residue between positions 98 and 99.

If this is the case the subtilase investigated is a subtilase within the scope of the present invention.

The alignment performed in step i) above is performed as described above by using the GAP routine.

Based on this description it is routine for a person skilled in the art to identify the active site loop (b) in a subtilase and determine if the subtilase in question is within the scope of the invention. If a variant is constructed by site directed mutagenesis, it is of course known beforehand if the subtilase variant is within the scope of the invention.

A subtilase variant of the invention may be constructed by standard techniques known in the art such as by site-directed/random mutagenesis or by DNA shuffling of different subtilase sequences. See section "PRODUCING A SUBTILASE VARIANT" and Material and methods herein (vide infra) for further details.

In further embodiments the invention relates to

1. an isolated subtilase enzyme according to the invention, wherein said at least one inserted amino acid residue is chosen from the group comprising: T,G,A, and S;

2. an isolated subtilase enzyme according to the invention, wherein said at least one inserted amino acid residue is chosen from the group of hydrophilic amino acid residues comprising:

C,N,Q,S and T, more preferably N,Q,S and T; 3. an isolated subtilase enzyme according to the invention, wherein said at least one inserted amino acid residue is chosen from the group of small hydrophobic amino acid residues comprising: A,G and V; or

4. an isolated subtilase enzyme according to the invention, wherein said at least one inserted amino acid residue is chosen from the group of large hydrophilic amino acid residues comprising: F.I.L.M,P,W and Y, more preferably F,I,L,M, and Y.

In a further embodiment, the invention relates to an isolated subtilase enzyme according to the invention, wherein said insertion between positions 98 and 99 comprises at least two amino acids, as compared to the corresponding active site loop in BLSAVI.

In further embodiments the invention relates co an isolated subtilase enzyme comprising at least one insertion, chosen from the group comprising (in BASBPN numbering):

X98X(T,G,A,S)
X98X{V, C,N, Q}
X98X{F,I,L,M,P,W,Y}

or more specific for subtilisin 309 and closely related subtilases, such as BAALKP, BLSUBL, and BSKSMK

A9BAA

A9AAT

A98AG

A9BAS

A98AV

A98AC

A98AN

A98AQ

A98AF

A98AI

A98AL

A98SD

A98TP

A98TW

A98AM

A98AP

A98AW

A98AY

Furthermore the invention relates to subtilases comprising the following multiple insertions in position 98

A98AGSGG
A98ATGSG
A98AGGGS
A98ASGSG
A98ATGTG
A98ASGTG
A98AGGGG
A98AGSGG

or any of the following combinations

A98ATD
A98ADT
G97D+A98AT
G97E+A98AT
G97K+A98AT
G97N+A98AT
G97Q+A98AT
G97R+A98AT
G97GD+A98AT
S87G+A98AGGGS
A98AT+S99SD
L96LD+A98AT
A98AT+Y167A
A98AT+R247K
A98GP+S99A
A98AS+A133E+T143K
A98AT+A108C+A138C
A98AT+Y167A+R170S+A194P
A98GI+S99H+G100S+S101A

It is well known in the art that a so-called conservative substitution of one amino acid residue to a similar amino acid residue is expected to produce only a minor change in the characteristic of the enzyme.

Table III below list groups of conservative amino acid substitutions.

Table III

Conservative amino acid substitutions

| Common Property | Amino Acid |
| --- | --- |
| Basic (positive charge) | K = lysine |
|  | H = histidine |
| Acidic (negative charge) | E = glutamic acid |
|  | D = aspartic acid |
| Polar | Q = glutamine |
|  | N = asparagine |
| Hydrophobic | L = leucine |
|  | I = isoleucine |

(continued)

Conservative amino acid substitutions

| Common Property | Amino Acid |
|---|---|
| | V = valine |
| | M = methionine |
| Aromatic | F = phenylalanine |
| | W = tryptophan |
| | Y = tyrosine |
| Small | G = glycine |
| | A = alanine |
| | S = serine |
| | T = threonine |

According to this principle subtilase variants comprising conservative substitutions, such as G97A+A98AS+S99G, G97S+A98AT+S99A are expected to exhibit characteristics that are not drastically different from each other.

Based on the disclosed and/or exemplified subtilase variants herein, it is routine work for a person skilled in the art to identify suitable conservative modification(s) to these variants in order to obtain other subtilase variants exhibiting similarly improved wash-performance.

According to the invention it the subtilases of the invention belong to the subgroups I-S1 and I-S2, especially subgroup I-S2, both for isolating novel enzymes of the invention from nature or from the artificial creation of diversity, and for designing and producing variants from a parent subtilase.

In relation to variants from subgroup I-S1, it is preferred to choose a parent subtilase from the group comprising BSS168 (BSSAS, BSAPRJ, BSAPRN, BMSAMP), BASBPN, BSSDY, BLSCAR (BLKERA, BLSCA1, BLSCA2, BLSCA3), BSSPRC, and BSSPRD, or functional variants thereof having retained the characteristic of sub-group I-S1.

In relation to variants from subgroup I-S2 it is preferred to choose a parent subtilase from the group comprising BSAPRQ, BLS147 (BSAPRM, BAH101), BLSAVI (BSKSMK, BAALKP, BLSUBL), BYSYAB, and BSAPRS, or functional variants thereof having retained the characteristic of sub-group I-S2.

In particular said parent subtilase is BLSAVI (SAVINASE® NOVO NORDISK A/S), and a preferred subtilase variant of the invention is accordingly a variant of SAVINASE®.

The present invention also comprises any of the above mentioned subtilases of the invention in combination with any other modification to the amino acid sequence thereof. Especially combinations with other modifications known in the art to provide improved properties to the enzyme are envisaged. The art describe a number of subtilase variants with different improved properties and a number of those are mentioned in the "Background of the invention" section herein *(vide supra).* Those references are disclosed here as references to identify a subtilase variant, which advantageously can be combined with a subtilase variant of the invention.

Such combinations comprise the positions: 222 (improve oxidation stability), 218 (improves thermal stability), substitutions in the Ca-binding sites stabilizing the enzyme, *e.g.* position 76, and many other apparent from the prior art.

In further embodiments a subtilase variant of the invention may advantageously be combined with one or more modification(s) in any of the positions:

27, 36, 57, 76, 87, 97, 101, 104, 120, 123, 167, 170, 206, 218, 222, 224, 235 and 274.

Specifically the following BLSAVI, BLSUBL, BSKSMK, and BAALKP variants are considered appropriate for combination:

K27R, *36D, S57P, N76D, S87N, G97N, S101G, S103A, V104A, V104I, V104N, V104Y, H120D, N123S, Y167, R170, Q206E, N218S, M222S, M222A, T224S, K235L and T274A.

Furthermore variants comprising any of the variants S101G+V104N, S87N+S101G+V104N, K27R+V104Y+N123S+T274A, N76D+S103A+V104I or N76D+V104A or other combinations of these mutations (V104N, S101G, K27R, V104Y, N123S, T274A, N76D, V104A) in combination with any one or more of the modification(s) mentioned above exhibit improved properties.

Even further subtilase variants of the main aspect(s) of the invention are preferably combined with one or more modification(s) in any of the positions 129, 131, 133 and 194, preferably as 129K, 131H, 133P, 133D and 194P modifications, and most preferably as P129K, P131H, A133P, A133D and A194P modifications. Any of those modification(s) are expected to provide a higher expression level of a subtilase variant of the invention in the production thereof.

Accordingly, an even further embodiment of the invention relates to a variant according to the invention, wherein said modification is chosen from the group comprising:

## PRODUCING A SUBTILASE VARIANT

Many methods for cloning a subtilase of the invention and for introducing insertions into genes (*e.g.* subtilase genes) are well known in the art, cf. the references cited in the "BACKGROUND OF THE INVENTION" section.

In general standard procedures for cloning of genes and introducing insertions (random and/or site directed) into said genes may be used in order to obtain a subtilase variant of the invention. For further description of suitable techniques reference is made to Examples herein (*vide infra*) and (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990); and WO 96/34946.

Further a subtilase variant of the invention may be constructed by standard techniques for artificial creation of diversity, such as by DNA shuffling of different subtilase genes (WO 95/22625; Stemmer WPC, Nature 370:389-91 (1994)). DNA shuffling of e.g. the gene encoding Savinase® with one or more partial subtilase sequences identified in nature to comprise an active site (b) loop regions longer than the active site (b) loop of Savinase®, will after subsequent screening for improved wash performance variants, provide subtilase variants according to the invention.

## EXPRESSION VECTORS

A recombinant expression vector comprising a DNA construct encoding the enzyme of the invention may be any vector which may conveniently be subjected to recombinant DNA procedures.

The choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one that on introduction into a host cell is integrated into the host cell genome in part or in its entirety and replicated together with the chromosome (s) into which it has been integrated.

The vector is preferably an expression vector in which the DNA sequence encoding the enzyme of the invention is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the enzyme.

The promoter may be any DNA sequence which shows transcriptional activity in the host cell of choice and may be derived from genes encoding proteins either homologous or heterologous to the host cell.

Examples of suitable promoters for use in bacterial host cells include the promoter of the *Bacillus stearothermophilus* maltogenic amylase gene, the *Bacillus licheniformis* alpha-amylase gene, the *Bacillus amyloliquefaciens* alpha-amylase gene, the *Bacillus subtilis* alkaline protease gen, or the *Bacillus pumilus* xylosidase gene, or the phage Lambda $P_R$ or $P_L$ promoters or the E. coli lac, trp or tac promoters.

The DNA sequence encoding the enzyme of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like kanamycin, chloramphenicol, erythromycin, tetracycline, spectinomycine, or the like, or resistance to heavy metals or herbicides.

To direct an enzyme of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al., op.cit.).

HOST CELL

The DNA sequence encoding the present enzyme introduced into the host cell may be either homologous or heterologous to the host in question. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell which is capable of producing the present enzyme and includes bacteria, yeast, fungi and higher eukaryotic cells including plants.

Examples of bacterial host cells which, on cultivation, are capable of producing the enzyme of the invention are gram-positive bacteria such as strains of *Bacillus,* such as strains of *B. subtilis, B. licheniformis, B. lentus, B. brevis, B. stearothermophilus, B. alkalophilus, B. amyloliquefaciens, B. coagulans, B. circulans, B. lautus, B. megatherium* or *B. thuringiensis,* or strains of *Streptomyces,* such as *S. lividans* or *S. murinus,* or gram-negative bacteria such as *Echerichia coli.*

The transformation of the bacteria may be effected by protoplast transformation, electroporation, conjugation, or by using competent cells in a manner known per se (cf. Sambrook et al., supra).

When expressing the enzyme in bacteria such as *E. coli,* the enzyme may be retained in the cytoplasm, typically as insoluble granules (known as inclusion bodies), or may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the enzyme in gram-positive bacteria such as Bacillus or Streptomyces strains, the enzyme may be retained in the cytoplasm, or may be directed to the extracellular medium by a bacterial secretion sequence. In the latter case, the enzyme may be recovered from the medium as described below.

METHOD OF PRODUCING SUBTILASE

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

When an expression vector comprising a DNA sequence encoding the enzyme is transformed into a heterologous host cell it is possible to enable heterologous recombinant production of the enzyme of the invention.

Thereby it is possible to make a highly purified subtilase composition, characterized in being free from homologous impurities.

In this context homologous impurities means any impurities (e.g. other polypeptides than the enzyme of the invention) which originate from the homologous cell where the enzyme of the invention is originally obtained from.

The medium used to culture the transformed host cells may be any conventional medium suitable for growing the host cells in question. The expressed subtilase may conveniently be secreted into the culture medium and may be recovered therefrom by well-known procedures including separating the cells from the medium by centrifugation or filtration, precipitating proteinaceous components of the medium by means of a salt such as ammonium sulfate, followed by chromatographic procedures such as ion exchange chromatography, affinity chromatography, or the like.

USE OF A SUBTILASE VARIANT OF THE INVENTION

A subtilase protease variant of the invention may be used for a number of industrial applications, in particular within the detergent industry.

Further the invention relates to an enzyme composition, which comprise a subtilase variant of the invention.

An summary of preferred industrial applications and corresponding preferred enzyme compositions are described below.

This summary is not in any way intended to be a complete list of suitable applications of a subtilase variant of the invention. A subtilase variants of the invention may be used in other industrial applications known in the art to include use of a protease, in particular a subtilase.

DETERGENT COMPOSITIONS COMPRISING THE MUTANT ENZYMES

The present invention comprises the use of the mutant enzymes of the invention in cleaning and detergent compositions and such compositions comprising the mutant subtilisin enzymes. Such cleaning and detergent compositions are well described in the art and reference is made to WO 96/34946; WO 97/07202; WO 95/30011 for further description of suitable cleaning and detergent compositions.

Furthermore the example(s) below demonstrate the improvements in wash performance for a number of subtilase variants of the invention.

Detergent Compositions

The enzyme of the invention may be added to and thus become a component of a detergent composition.

The detergent composition of the invention may for example be formulated as a hand or machine laundry detergent compo-sition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase™, Savinase™, Primase™, Duralase™, Esperase™, and Kannase™ (Novo Nordisk A/S), Maxatase™, Maxacal™, Maxapem™, Properase™, Purafect™, Purafect OxP™, FN2™, and FN3™ (Genencor International Inc.).

Lipases: Suitable lipases include those of bacterial or fungal origin. Chemically modified or protein engineered mutants are included. Examples of useful lipases include lipases from *Humicola* (synonym *Thermomyces),* e.g. from *H. lanuginosa (T. lanuginosus)* as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase™ and Lipolase Ultra™ (Novo Nordisk A/S).

Amylases: Suitable amylases (α and/or β) include those of bacterial or fungal origin. Chemically modified or protein engineered mutants are included. Amylases include, for example, α-amylases obtained from *Bacillus,* e.g. a special strain of *B. licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novo Nordisk A/S), Rapidase™ and Purastar™ (from Genencor International Inc.).

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from Humicola insolens, *Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novo Nordisk A/S), Clazinase™, and Puradax HA™ (Genencor International Inc.), and KAC-500(B)™ (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g. from *C. cinereus*, and variants thereof as those described in WO 93/24618, WO 95/10602, and WO 98/15257.

Commercially available peroxidases include Guardzyme™ (Novo Nordisk A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or nonaqueous.

The detergent composition comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl R1-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-100 mg of enzyme protein per liter of wash liquor, preferably 0.05-5 mg of enzyme protein per liter of wash liquor, in particular 0.1-1 mg of enzyme protein per liter of wash liquor.

The enzyme of the invention may additionally be incorporated in the detergent formulations disclosed in WO 97/07202 which is hereby incorporated as reference.

LEATHER INDUSTRY APPLICATIONS

A subtilase of the invention may be used in the leather industry, in particular for use in depilation of skins.
In said application a subtilase variant of the invention is preferably used in an enzyme composition which further comprises another protease.
For a more detailed description of suitable other proteases see section relating to suitable enzymes for use in a detergent composition *(vide supra).*

WOOL INDUSTRY APPLICATIONS

A subtilase of the invention may be used in the wool industry, in particular for use in cleaning of clothes comprising wool.
In said application a subtilase variant of the invention is preferably used in an enzyme composition which further comprises another protease.
For a more detailed description of suitable other proteases see section relating to suitable enzymes for use in a detergent composition (*vide supra).*
The invention is described in further detail in the following examples which are not in any way intended to limit the scope of the invention as claimed.

**MATERIALS AND METHODS**

STRAINS:

*B. subtilis* DN1885 (Diderichsen et al., 1990).
*B. lentus* 309 and 147 are specific strains of *Bacillus lentus*, deposited with the NCIB and accorded the accession numbers NCIB 10309 and 10147, and described in US Patent No. 3,723,250 incorporated by reference herein.
*E. coli* MC 1000 (M.J. Casadaban and S.N. Cohen (1980); *J. Mol. Biol.* **138** 179-207), was made r⁻,m⁺ by conventional methods and is also described in US Patent Application Serial No. 039,298.

PLASMIDS:

pJS3: *E. coli - B. subtilis* shuttle vector containing a synthetic gene encoding for subtilase 309. (Described by Jacob Schiødt et al. in Protein and Peptide letters 3:39-44 (1996}).
pSX222: *B. subtilis* expression vector (Described in WO 96/34946).

GENERAL MOLECULAR BIOLOGY METHODS:

Unless otherwise mentioned the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).
Enzymes for DNA manipulations were used according to the specifications of the suppliers.

ENZYMES FOR DNA MANIPULATIONS

Unless otherwise mentioned all enzymes for DNA manipulations, such as *e.g.* restiction endonucleases, ligases etc., are obtained from New England Biolabs, Inc.

PROTEOLYTIC ACTIVITY

In the context of this invention proteolytic activity is expressed in Kilo NOVO Protease Units (KNPU). The activity is determined relatively to an enzyme standard (SAVINASE®), and the determination is based on the digestion of a dimethyl casein (DMC) solution by the proteolytic enzyme at standard conditions, i.e. 50°C, pH 8.3, 9 min. reaction time, 3 min. measuring time. A folder AF 220/1 is available upon request to Novo Nordisk A/S, Denmark, which folder is hereby included by reference.

A GU is a Glycine Unit, defined as the proteolytic enzyme activity which, under standard conditions, during a 15 minutes' incubation at 40°C, with N-acetyl casein as substrate, produces an amount of $NH_2$-group equivalent to 1 mmole of glycine.

Enzyme activity can also be measured using the PNA assay, according to reaction with the soluble substrate succinyl-alanine-alanine-proline-phenyl-alanine-para-nitro-phenol, which is described in the Journal of American Oil Chemists Society, Rothgeb, T.M., Goodlander, B.D., Garrison, P.H., and Smith, L.A., (1988).

FERMENTATION:

Fermentations for the production of subtilase enzymes were performed at 30°C on a rotary shaking table (300 r.p.m.) in 500 ml baffled Erlenmeyer flasks containing 100 ml BPX medium for 5 days.

Consequently in order to make an e.g. 2 liter broth 20 Erlenmeyer flasks were fermented simultaneously.

MEDIA:

| BPX Medium Composition (per liter) | |
|---|---|
| Potato starch | 100 g |
| Ground barley | 50 g |
| Soybean flour | 20 g |
| $Na_2HPO_4 \times 12\,H_2O$ | 9 g |
| Pluronic | 0.1 g |
| Sodium caseinate | 10 g |

The starch in the medium is liquefied with α-amylase and the medium is sterilized by heating at 120°C for 45 minutes. After sterilization the pH of the medium is adjusted to 9 by addition of $NaHCO_3$ to 0.1 M.

**EXAMPLE 1**

CONSTRUCTION AND EXPRESSION OF ENZYME VARIANTS: SITE-DIRECTED MUTAGENESIS:

Subtilase 309 site-directed variants of the invention comprising specific insertions in the active site loop (b) between positions 98 and 99 were made by traditional cloning of DNA fragments (Sambrook et al., Molecular Cloning: A Laboratory Manual, 2nd Ed., Cold Spring Harbor, 1989) produced by PCR of oligos containing the desired insertions (see below).

The template plasmid DNA was pJS3, or an analogue of this containing a variant of Subtilase 309._

Insertions were introduced by oligo directed mutagenesis to the construction of A98AX insertion variants (X = any amino acid residue inserted between positions 98 and 99) resulting in A98AX subtilase 309 variants.

The Subtilase 309 variants were transformed into *E. coli.* DNA purified from a over night culture of these transformants were transformed into *B. subtilis* by restriction endonuclease digestion, purification of DNA fragments, ligation, transformation of *B. subtilis.* Transformation of B. subtilis was performed as described by Dubnau et al., 1971, J. Mol. Biol. 56, pp. 209-221.

LOCALIZED RANDOM MUTAGENESIS IN ORDER TO INSERT RANDOM INSERTIONS IN A LOCALIZED REGION:

The overall strategy used to perform localized random mutagenesis was:

A mutagenic primer (oligonucleotide) was synthesized, that corresponds to the DNA sequence flanking the site of insertion, separated by the DNA base pairs defining the insertion.

Subsequently, the resulting mutagenic primer was used in a PCR reaction with a suitable opposite primer. The resulting PCR fragment was purified and extended in a second PCR-reaction, before being digested by endonucleases and cloned into the *E. coli - B. subtilis* shuttle vector (see below).

Alternatively, and if necessary, the resulting PCR fragment is used in a second PCR reaction as a primer with a second suitable opposite primer to allow digestion and cloning of the mutagenized region into the shuttle vector. The PCR reactions are performed under normal conditions.

Following this strategy a localized random library was constructed in SAVINASE wherein insertions were introduced in the active site loop region between positions 98 and 99.

The mutations were introduced by mutagenic primers (see below), so that all 20 amino acids are represented (N = 25% of A, T, C, and G; whereas S = 50% C and G. The produced PCR fragment was extended towards the N-terminal of Savinase by another round of PCR by combination of a overlapping sequence with a PCR-fragment produced by PCR-amplification with primers; 5' CTA AAT ATT CGT GGTGGC GC 3' *(sense)* and 5' GAC TTT AAC AGC GTA TAG CTC AGC 3' *(antisense)*. The extended DNA-fragments were cloned into the Hind III- and Mlu I- sites of the modified plasmid pJS3 (see above), and ten randomly chosen *E. coli* colonies were sequenced to confirm the mutations designed.

The mutagenic primer (5' GCT GAG CTA TAC GCT GTT AAA GTC CTA GGG GCG NNS AGC GGT TCA GGT TCG TCA AGC *3'(sense))* was used in a PCR reaction with a suitable *anti-sense* opposite primer, situated downstream of the Mlu I site in pJS3 (e.g. 5'- CCC TTT AAC CGC ACA GCG TTT -3' *(anti-sense))* and the plasmid pJS3 as template. This resulting PCR product was cloned into the pJS3 shuttle vector by using the restriction enzymes Hind III and Mlu I.

The random library was transformed into E. coli by well known techniques.

The library prepared contained approximately 100,000 individual clones/library.

Ten randomly chosen colonies were sequenced to confirm the mutations designed.

In order to purify a subtilase variant of the invention, the *B. subtilis* pJS3 expression plasmid comprising a variant of the invention was transformed into a competent *B. subtilis* strain and was fermented as described above in a medium containing 10 μg/ml Chloramphenicol (CAM).

## EXAMPLE 2

### PURIFICATION OF ENZYME VARIANTS:

This procedure relates to purification of a 2 liter scale fermentation for the production of the subtilases of the invention in a *Bacillus* host cell.

Approximately 1.6 liters of fermentation broth were centrifuged at 5000 rpm for 35 minutes in 1 liter beakers. The supernatants were adjusted to pH 6.5 using 10% acetic acid and filtered on Seitz Supra S100 filter plates.

The filtrates were concentrated to approximately 400 ml using an Amicon CH2A UF unit equipped with an Amicon S1Y10 UF cartridge. The UF concentrate was centrifuged and filtered prior to absorption at room temperature on a Bacitracin affinity column at pH 7. The protease was eluted from the Bacitracin column at room temperature using 25% 2-propanol and 1 M sodium chloride in a buffer solution with 0.01 dimethylglutaric acid, 0.1 M boric acid and 0.002 M calcium chloride adjusted to pH 7.

The fractions with protease activity from the Bacitracin purification step were combined and applied to a 750 ml Sephadex G25 column (5 cm dia.) equilibrated with a buffer containing 0.01 dimethylglutaric acid, 0.2 M boric acid and 0.002 m calcium chloride adjusted to pH 6.5.

Fractions with proteolytic activity from the Sephadex G25 column were combined and applied to a 150 ml CM Sepharose CL 6B cation exchange column (5 cm dia.) equilibrated with a buffer containing 0.01 M dimethylglutaric acid, 0.2 M boric acid, and 0.002 M calcium chloride adjusted to pH 6.5.

The protease was eluted using a linear gradient of 0-0.1 M sodium chloride in 2 litres of the same buffer (0-0.2 M sodium chloride in case of Subtilisin 147).

In a final purification step protease containing fractions from the CM Sepharose column were combined and concentrated in an Amicon ultrafiltration cell equipped with a GR81PP membrane (from the Danish Sugar Factories Inc.).

By using the techniques of Example 1 for the construction and fermentation, and the above isolation procedure the following subtilisin 309 variants were produced and isolated:

A98AT
A98AS
A98AD
A98AE
A98AP
A98AG
A98AH
A98AI
A98AT+Y167A
A98AD

A98AG
A98AH
A98AI
A98AN
A98AP
A98AS
A98AT
A98AV
A98AY
A98SD
A98TP
A98TW
A98ASGTG
A98ATGSG
A98ATGTG
A98AGGGG
A98AGSGG
A98AT+Y167A
A98AT+R247K
A98GP+S99A
G97D+A98AT
G97E+A98AT
G97K+A98AT
G97N+A98AT
G97Q+A98AT
G97R+A98AT
S87G+A98AGGGS
A98AS+A133E+T143K
A98AT+A108C+A138C
A98AT+Y167A+R170S+A194P
A98GI+S99H+G100S+S101A

These variants were found to exhibit better wash performance than Savinase in a preliminary assay.

**EXAMPLE 3**

WASH PERFORMANCE OF DETERGENT COMPOSITIONS COMPRISING ENZYME VARIANTS

The following examples provide results from a number of washing tests that were conducted under the conditions indicated

MINI WASH

| WASH CONDITIONS: | | |
|---|---|---|
| | Europe | US |
| Detergent Dosage | 4g/l | 1g/l |
| Wash Temp | 30°C | 25°C |
| Wash Time | 30min | 10min |
| Water hardness | 18°dH ($Ca^{2+}$/$Mg^{2+}$ =5:1) | 6°dH ($Ca^{2+}$/$Mg^{2+}$= 2:1) |
| Ph | Not adjusted | Not adjusted |
| Enzyme conc. | 1, 2, 5, 10, 30 nM | 1, 2, 5, 10, 30 nM |

(continued)

| WASH CONDITIONS: | | |
|---|---|---|
| | Europe | US |
| Test system | 150 ml glass beakers with a stirring rod | 150 ml glass beakers with a stirring rod |
| Textile/volume | 5 textile pieces (Ø 2.5 cm) in 50 ml detergent | 5 textile pieces (Ø 2.5 cm) in 50 ml detergent |
| Test Material | EMPA116 | EMPA117 |

DETERGENTS:

The detergents used were obtained from supermarkets in Denmark (OMO, datasheet ED-9745105) and the USA (Wisk, datasheet ED-9711893), respectively. Prior to use all enzymatic activity in the detergents was inactivated by micro wave treatment.

SWATCHES;

The swatches used were EMPA116 and EMPA117, obtained from EMPA Testmaterialen, Movenstrasse 12, CH-9015 St. Gall, Switzerland.

REFLECTANCE

Measurement of reflectance (R) on the test material was done at 460 nm using a Macbeth ColorEye 7000 photometer. The measurements were done according to the manufacturers protocol.

EVALUATION

The evaluation of the wash performance of a subtilase is determined by either the improvement factor or the performance factor for the subtilase iinvestigated.
The improvement factor, $IF_{Dose/response}$, is defined as the ratio between the slopes of the wash performance curves for a detergent containing the subtilase investigated and the same detergent containing a reference subtilase at the asymptotic concentration of the subtilase goes to zero

$$IF_{Dose/response} = a/a_{ref}$$

The wash performance is calculated according to the formula I:

$$R = R_0 + \frac{a \cdot \Delta R_{max} \cdot c}{\Delta R_{max} + a \cdot c} \qquad (I);$$

where
R is the wash performance in reflectance units; $R_0$ is the intercept of the fitted curve with y-axis (blind); a is the slope of the fitted curve as $c \to 0$; c is the enzyme concentration; and $\Delta R_{max}$ is the theoretical maximal wash effect as $c \to \infty$.
The performance factor, P, is calculated according to the formula II

$$P = \frac{R_{Variant} - R_{Blank}}{R_{Savinase} - R_{Blank}} \qquad (II)$$

where
$R_{variant}$ is the reflectance of test material washed with 10nM variant; $R_{savinase}$ is the reflectance of test material washed with 10nM Savinase; $R_{blank}$ is the reflectance of test material washed with no enzyme
US (detergent: US Wisk, Swatch: EMPA117)

| Variant | IFDose/response | P |
|---|---|---|
| A98AT | >3 | 2.3 |
| A98AD | | 1.2 |
| A98AG | | 1.1 |
| A98AH | | 1.4 |
| A98AI | | 1.2 |
| A98AN | | 1.0 |
| A98AP | | 1.3 |
| A98AS | | 1.6 |
| A98AV | | 1.1 |
| A98AY | | 1.1 |
| A98SD | | 1.2 |
| A98TP | | 1.5 |
| A98TW | | 1.2 |
| A98AGGGG | | 1.1 |
| A98ASGTG | | 1.1 |
| A98ATGSG | | 1.3 |
| A98ATGTG | | 1.2 |
| A98AGSGG | ---- | 1.7 |
| A98AT+Y167A | | 1.7 |
| A98AT+R247K | | 1.3 |
| A98GP+S99A | | 1.1 |
| G97D+A98AT | | 1.4 |
| G97E+A98AT | | 1.6 |
| G97K+A98AT | | 1.0 |
| G97N+A98AT | | 1.2 |
| G97Q+A98AT | | 1.0 |
| G97R+A98AT | | 1.0 |
| S87G+A98AGGGS | | 1.2 |
| A98AS+A133E+T143K | | 0.8 |
| A98AT+A108C+A138C | | 1.2 |
| A98AT+Y167A+R170S+A194P | | 1.3 |
| A98GI+S99H+G100S+S101A | | 1.0 |

The subtilases of the inventions are thus seen to exhibit improved wash performance in comparison to Savinase®.

## Claims

1. An isolated subtilase enzyme of the I-S1 and I-S2 sub-groups having at least one additional amino acid residue in position 98 of the active site loop (b) region from position 95 to 103, whereby said additional amino acid residue(s) correspond to the insertion of at least one amino acid residue between positions 98 and 99 wherein said at least one additional or inserted amino acid residue is chosen from the group consisting of: G,N,I,S,T,C,Q,V,P,A,L,M,P, W and Y.

2. The isolated subtilase enzyme according to claim 1, wherein said at least one additional or inserted amino acid residue, comprises more than one additional or inserted amino acid residue in the active site loop (b).

3. The isolated subtilase enzyme of claim 1 or 2, wherein said insertion(s) between positions 98 and 99 are combined with one or more further modification(s) in any other position(s).

4. The isolated subtilase enzyme of claim 3, wherein said further modification(s) are in one or more of the positions 27, 36, 57, 76, 87, 97, 101, 104, 120, 123, 167, 170, 206, 218, 222, 224, 235 and 274.

5. The isolated subtilase enzyme of any of the preceding claims, wherein said modification(s) is/are combined with modification(s) in one or more of the positions 129, 131, 133 and 194.

6. The isolated subtilase enzyme of any of the preceding claims, wherein the subtilase belongs to the sub-group I-S1.

7. The isolated subtilase enzyme of claim 6, which is chosen from the group consisting of BSS168, BASBPN, BSSDY and BLSCAR.

8. The isolated subtilase enzyme according to any of claims 1-5, wherein the subtilase belongs to the sub-group I-S2.

9. The isolated subtilase enzyme of claim 8, which is chosen from the group consisting of BLS147, BLS309, BAALKP and BYSYAB.

10. The isolated subtilase enzyme of claim 1, 8 or 9, which enzyme comprises an insertion selected from the group consisting of

A98AA,
A98AT,
A98AG,
A98AS,
A98AV,
A98AC,
A9BAN,
A98AQ,
A98AF,
A98AI,
A98AL,
A98AM,
A98AP,
A98AW,
A98AY,
A98SD,
A98TP and
A98TW.

11. The isolated subtilase enzyme of any of the claims 8-10, wherein said further modification(s) are chosen from the group consisting of K27R, *36D, S57P, N76D, S87N, G97N, S101G, V104A, V104N, V104Y, H120D, N123S, Y167X, R170X, Q206E, N216S, M222S, M222A, T224S, K235L, and T274A.

12. The isolated subtilase enzyme of any of the claims 8-10, wherein said further modification(s) are chosen from the group consisting of S101G+V104N, S87N+S101G+V104N, K27R+V104Y+N123S+T274A, N76D+S103A+V104I or N76D+V104A, or other combinations of these mutations (V104N, S101G, K27R, V104Y, N123S, T274A, N76D, V104A), in combination with any one or more of the substitutions and/or insertions mentioned in any of the preceding claims.

13. The isolated subtilase enzyme of any of the claims 8-10, wherein said further modification(s) are chosen from the group further consisting of P129K, P131H, A133P, A133D and A194P.

14. The isolated subtilase enzyme according to any of the preceding claims comprising the modification chosen from the group consisting of: A98AT+Y167A; A98AT+S99SD; A98ATD; A98ADT A98AGSGG; A98ATGSG; A98AGGGS; A98AGGGG; A98ASGTG; A98ATGTG; S87G+A98AGGGS; L96LD+ A9BAT; G97D+A98AT; C97E+A98AT; G97K+A98AT; G97N+A98AT; G97Q+A98AT; G97R+A98AT; G97GD+A9BAT; A9BAT+Y167A; A98AT+R247K; A98GP+S99A; A98AS+A133E+T143K; A98AT+A108C+A138C; A98AT+Y167A+R170S+A194P; A98GI+S99H+G100S+S101A.

15. The isolated subtilase enzyme of claim 1, which enzyme is belonging to the I-S1 sub-group having the amino acid sequence:

```
    1                  10                 20                 30
A-Q-T-V-P-Y-G-I-P-L-I-K-A-D-K-V-Q-A-Q-G-F-K-G-A-N-V-K-V-A-V
                       40                 50                 60
L-D-T-G-I-Q-A-S-H-P-D-L-N-V-V-G-G-A-S-F-V-A-G-E-A-*-Y-N-T-D


                       70                 80                 90
G-N-G-H-G-T-H-V-A-G-T-V-A-A-L-D-N-T-T-G-V-L-G-V-A-P-S-V-S-L
                       98a                110                120
Y-A-V-K-V-L-N-S-X-S-G-S-G-T-Y-S-G-I-V-S-G-I-E-W-A-T-T-N-G-M-D
                       130                140                150
V-I-N-M-S-L-G-G-P-S-G-S-T-A-M-K-Q-A-V-D-N-A-Y-A-R-G-V-V-V-V
                       160                170                180
A-A-A-G-N-S-G-S-S-G-N-T-N-T-I-G-Y-P-A-K-Y-D-S-V-I-A-V-G-A-V
                       190                200                210
D-S-N-S-N-R-A-S-F-S-S-V-G-A-E-L-E-V-M-A-P-G-A-G-V-Y-S-T-Y-P
                       220                230                240
T-S-T-Y-A-T-L-N-G-T-S-M-A-S-P-H-V-A-G-A-A-A-L-I-L-S-K-H-P-N
                       250                260                270
L-S-A-S-Q-V-R-N-R-L-S-S-T-A-T-Y-L-G-S-S-F-Y-Y-G-K-G-L-I-N-V
        275
E-A-A-A-Q
```

16. The isolated subtilase enzyme of claim 1, which enzyme is belonging to the I-S2 sub-group having the amino acid sequence:

```
 .1                    10                  20                  30
 .A-Q-S-V-P-W-G-I-S-R-V-Q-A-P-A-A-H-N-R-G-L-T-G-S-G-V-K-V-A-V-
 .                     40                  50                  60
 .L-D-T-G-I-*-S-T-H-P-D-L-N-I-R-G-G-A-S-F-V-P-G-E-P-*-S-T-Q-D-
 .                     70                  80                  90
 .G-N-G-H-G-T-H-V-A-G-T-I-A-A-L-N-N-S-I-G-V-L-G-V-A-P-S-A-E-L-
 .                     98a                 110                 120
 .Y-A-V-K-V-L-G-A-X-S-G-S-G-S-V-S-S-I-A-Q-G-L-E-W-A-G-N-N-G-M-H-
 .                     130                 140                 150
 .V-A-N-L-S-L-G-S-P-S-P-S-A-T-L-E-Q-A-V-N-S-A-T-S-R-G-V-L-V-V-


 .                     160                 170                 180
 .A-A-S-G-N-S-G-A-*-G-S-I-S-*-*-*-Y-P-A-R-Y-A-N-A-M-A-V-G-A-T-
 .                     190                 200                 210
 .D-Q-N-N-N-R-A-S-F-S-Q-Y-G-A-G-L-D-I-V-A-P-G-V-N-V-Q-S-T-Y-P-
 .                     220                 230                 240
 .G-S-T-Y-A-S-L-N-G-T-S-M-A-T-P-H-V-A-G-A-A-A-L-V-K-Q-K-N-P-S-
 .                     250                 260                 270
 .W-S-N-V-Q-I-R-N-H-L-K-N-T-A-T-S-L-G-S-T-N-L-Y-G-S-G-L-V-N-A-
 .                     275
 .E-A-A-T-R
```

**17.** The isolated subtilase enzyme of claims 15 or 16, wherein X in position 98a is chosen from the group consisting of T, A, G, S and P.

**18.** An isolated DNA sequence encoding a subtilase enzyme of any of the preceding claims.

**19.** An expression vector comprising an isolated DNA sequence of claim 18.

**20.** A microbial host cell transformed with an expression vector of claim 19.

**21.** The microbial host of claim 20, which is a bacterium, preferably a Bacillus, especially *B. lentus*.

**22.** The microbial host of claim 20, which is a fungus or yeast, preferably a filamentous fungus, especially an Aspergillus.

**23.** A method for producing an isolated subtilase enzyme of any of claims 1 to 17, wherein a host of any of claims 20-22 is cultured under conditions conducive to the expression and secretion of said subtilase enzyme, and the subtilase enzyme is recovered.

**24.** A composition comprising an isolated subtilase enzyme according to any of claims 1 to 17.

**25.** The composition according to claim 24, which additionally comprises a cellulase, lipase, cutinase, oxidoreductase, another protease, or an amylase.

26. The composition according to claim 24 or 25, wherein the composition is a detergent composition.

27. Use of an isolated subtilase enzyme according to any of claims 1 to 17 or an enzyme composition according to claims 24 or 25 in a laundry and/or a dishwash detergent.

**Patentansprüche**

1. Isoliertes Subtilaseenzym der Untergruppen I-S1 und I-S2, welches mindestens einen zusätzlichen Aminosäurerest an Position 98 der Schleife (Loop) (b) der Region des aktiven Zentrums von Position 95 bis 103 aufweist, wobei der/die zusätzliche(n) Aminosäurerest(e) der Insertion von mindestens einem Aminosäurerest zwischen den Positionen 98 und 99 entspricht / entsprechen, wobei der mindestens eine zusätzliche oder eingefügte Aminosäurerest ausgewählt ist aus der Gruppe, bestehend aus: G, N, I, S, T, C, Q, V, F, A, L, M, P, W und Y.

2. Isoliertes Subtilaseenzym nach Anspruch 1, wobei der mindestens eine zusätzliche oder eingefügte Aminosäurerest mehr als einen zusätzlichen oder eingefügten Aminosäurerest in der Schleife (b) des aktiven Zentrums umfasst.

3. Isoliertes Subtilaseenzym nach Anspruch 1 oder 2, wobei die Insertion(en) zwischen den Positionen 98 und 99 mit einer oder mehreren weiteren Modifikation(en) an einer beliebigen / beliebigen anderen Position(en) kombiniert ist / sind.

4. Isoliertes Subtilaseenzym nach Anspruch 3, wobei die weitere(n) Modifikation(en) an einer oder mehreren der Positionen 27, 36, 57, 76, 87, 97, 101, 104, 120, 123, 167, 170, 206, 218, 222, 224, 235 und 274 vorliegt / vorliegen.

5. Isoliertes Subtilaseenzym nach einem beliebigen der vorangehenden Ansprüche, wobei die Modifikation(en) mit Modifikation(en) an einer oder mehreren der Positionen 129, 131, 133 und 194 kombiniert ist / sind.

6. Isoliertes Subtilaseenzym nach einem beliebigen der vorangehenden Ansprüche, wobei die Subtilase der Untergruppe I-S1 angehört.

7. Isoliertes Subtilaseenzym nach Anspruch 6, welches ausgewählt ist aus der Gruppe bestehend aus BSS168, BASBPN, BSSDY und BLSCAR.

8. Isoliertes Subtilaseenzym nach einem beliebigen der Ansprüche 1 bis 5, wobei die Subtilase der Untergruppe I-S2 angehört.

9. Isoliertes Subtilaseenzym nach Anspruch 8, welches ausgewählt ist aus der Gruppe bestehend aus BLS147, BLS309, BAALKP und BYSYAB.

10. Isoliertes Subtilaseenzym nach Anspruch 1, 8 oder 9, welches Enzym eine Insertion umfasst, ausgewählt aus der Gruppe, bestehend aus

A98AA,
A98AT,
A9BAG,
A98AS,
A98AV,
A98AC,
A98AN,
A9BAQ,
A98AF,
A98AI,
A98AL,
A98AM,
A98AP,
A98AW,
A98AY,
A98SD,

A98TP und
A98TW.

**11.** Isoliertes Subtilaseenzym nach einem beliebigen der Ansprüche 8 bis 10, wobei die weitere(n) Modifikation(en) ausgewählt ist / sind aus der Gruppe bestehend aus K27R, *36D, S57P, N76D, S87N, G97N, S101G, V104A, V104N, V104Y, H120D, N123S, Y167X, R170X, Q206E, N218S, M222S, M222A, T224S, K235L und T274A.

**12.** Isoliertes Subtilaseenzym nach einem beliebigen der Ansprüche 8 bis 10, wobei die weitere(n) Modifikation(en) ausgewählt ist / sind aus der Gruppe bestehend aus S101G+V104N, S87N+S101G+V104N, K27R+V104Y+N123S+T274A, N76D+S103A+V104I oder N76D+V104A oder anderen Kombinationen dieser Mutationen (V104N, S101G, K27R, V104Y, N123S, T274A, N76D, V104A) in Kombination mit einer beliebigen oder mehreren beliebigen der Substitutionen und/oder Insertionen, die in einem beliebigen der vorangehenden Ansprüche erwähnt ist / sind.

**13.** Isoliertes Subtilaseenzym nach einem beliebigen der Ansprüche 8 bis 10, wobei die weitere(n) Modifikation(en) ausgewählt ist / sind aus der Gruppe, die weiterhin aus P129K, P131H, A133P, A133D und A194P besteht.

**14.** Isoliertes Subtilaseenzym nach einem beliebigen der vorangehenden Ansprüche, welches die Modifikation, ausgewählt aus der Gruppe bestehend aus:

A98AT+Y167A; A98AT+899SD; A98ATD; A98ADT A98AGSGG; A98ATGSG; A98AGGGS; A98AGGGG; A98ASGTG; A98ATGTG, S87G+A98AGGGS; L96LD+ A98AT; G97D+A98AT; G97E+A98AT; G97K+A98AT; G97N+A98AT; G97Q+A98AT; G97R+A98AT; G97GD+A98AT; A98AT+Y167A; A98AT+RZ47K; A98GP+S99A; A98A8+A133E+T143K; A98AT+A108C+A138C; A98AT+Y167A+R170S+A194P; A98GI+S99H+G100S+S101A

umfasst.

**15.** Isoliertes Subtilaseenzym nach Anspruch 1, welches Enzym der Untergruppe I-S1 angehört, welches die Aminosäuresequenz aufweist:

```
1               10              20              30
A-Q-T-V-P-Y-G-I-P-L-I-K-A-D-K-V-Q-A-Q-G-F-K-G-A-N-V-K-V-A-V
                40              50              60
L-D-T-G-I-Q-A-S-H-P-D-L-N-V-V-G-G-A-S-F-V-A-G-E-A-*-Y-N-T-D
```

```
                              70                    80                    90
          G-N-G-H-G-T-H-V-A-G-T-V-A-A-L-D-N-T-T-G-V-L-G-V-A-P-S-V-S-L
                             98a                   110                   120
          Y-A-V-K-V-L-N-S-X-S-G-S-G-T-Y-S-G-I-V-S-G-I-E-W-A-T-T-N-G-M-D
                             130                   140                   150
          V-I-N-M-S-L-G-G-P-S-G-S-T-A-M-K-Q-A-V-D-N-A-Y-A-R-G-V-V-V-V
                             160                   170                   180
          A-A-A-G-N-S-G-S-S-G-N-T-N-T-I-G-Y-P-A-K-Y-D-S-V-I-A-V-G-A-V
                             190                   200                   210
          D-S-N-S-N-R-A-S-F-S-S-V-G-A-E-L-E-V-M-A-P-G-A-G-V-Y-S-T-Y-P
                             220                   230                   240
          T-S-T-Y-A-T-L-N-G-T-S-M-A-S-P-H-V-A-G-A-A-A-L-I-L-S-K-H-P-N
                             250                   260                   270
          L-S-A-S-Q-V-R-N-R-L-S-S-T-A-T-Y-L-G-S-S-F-Y-Y-G-K-G-L-I-N-V
                             275
          E-A-A-A-Q
```

**16.** Isoliertes Subtilaseenzym nach Anspruch 1, welches Enzym der Untergruppe I-S2 angehört, welches die Amino-säuresequenz aufweist:

```
 1                10                  20                  30
A-Q-S-V-P-W-G-I-S-R-V-Q-A-P-A-A-H-N-R-G-L-T-G-S-G-V-K-V-A-V-
                 40                  50                  60
L-D-T-G-I-*-S-T-H-P-D-L-N-I-R-G-G-A-S-F-V-P-G-E-P-*-S-T-Q-D-
                 70                  80                  90
G-N-G-H-G-T-H-V-A-G-T-I-A-A-L-N-N-S-I-G-V-L-G-V-A-P-S-A-E-L-
                 98a                 110                 120
Y-A-V-K-V-L-G-A-X-S-G-S-G-S-V-S-S-I-A-Q-G-L-E-W-A-G-N-N-G-M-H-
                 130                 140                 150
V-A-N-L-S-L-G-S-P-S-P-S-A-T-L-E-Q-A-V-N-S-A-T-S-R-G-V-L-V-V-

                 160                 170                 180
A-A-S-G-N-S-G-A-*-G-S-I-S-*-*-*-Y-P-A-R-Y-A-N-A-M-A-V-G-A-T-
                 190                 200                 210
D-Q-N-N-N-R-A-S-F-S-Q-Y-G-A-G-L-D-I-V-A-P-G-V-N-V-Q-S-T-Y-P-
                 220                 230                 240
G-S-T-Y-A-S-L-N-G-T-S-M-A-T-P-H-V-A-G-A-A-A-L-V-K-Q-K-N-P-S-
                 250                 260                 270
W-S-N-V-Q-I-R-N-H-L-K-N-T-A-T-S-L-G-S-T-N-L-Y-G-S-G-L-V-N-A-
                 275
E-A-A-T-R
```

**17.** Isoliertes Subtilaseenzym nach Anspruch 15 oder 16, wobei X an Position 98a ausgewählt ist aus der Gruppe bestehend aus T, A, G, S und P.

**18.** Isolierte DNA-Sequenz, welche ein Subtilaseenzym nach einem beliebigen der vorangehenden Ansprüche kodiert.

**19.** Expressionsvektor, welcher eine isolierte DNA-Sequenz nach Anspruch 18 umfasst.

**20.** Mikrobielle Wirtszelle, die mit einem Expressionsvektor nach Anspruch 19 transformiert ist.

**21.** Mikrobieller Wirt nach Anspruch 20, welcher ein Bakterium, bevorzugt ein Bacillus, besonders *B. lentus,* ist.

**22.** Mikrobieller Wirt nach Anspruch 20, welcher ein Pilz oder Hefe, bevorzugt ein filamentöser Pilz, besonders ein Aspergillus, ist.

**23.** Verfahren zum Herstellen eines isolierten Subtilaseenzyms nach einem beliebigen der Ansprüche 1 bis 17, wobei ein Wirt nach einem beliebigen der Ansprüche 20 bis 22 unter Bedingungen kultiviert wird, die für die Expression und Sekretion des Subtilaseenzyms zuträglich sind, und das Subtilaseenzym gewonnen wird.

**24.** Zusammensetzung, welche ein isoliertes Subtilaseenzym nach einem beliebigen der Ansprüche 1 bis 17 umfasst.

**25.** Zusammensetzung nach Anspruch 24, welche zusätzlich eine Cellulase, Lipase, Cutinase, Oxidoreductase, eine andere Protease oder eine Amylase umfasst.

**26.** Zusammensetzung nach Anspruch 24 oder 25, wobei die Zusammensetzung eine Detergenszusammensetzung ist.

27. Verwendung eines isolierten Subtilaseenzyms nach einem beliebiegen der Ansprüche 1 bis 17 oder einer Enzymzusammensetzung nach Anspruch 24 oder 25 in einem Wasch- und/oder einem Geschirrspüldetergens.

**Revendications**

1. Enzyme subtilase isolée des sous-groupes I-S1 et I-S2 ayant au moins un résidu d'acide aminé supplémentaire en position 98 de la région en boucle (b) du site actif de la position 95 à 103, ledit(lesdits) résidu(s) d'acide(s) aminé (s) supplémentaire (s) correspondant à l'insertion d'au moins un résidu d'acide aminé entre les positions 98 et 99, dans laquelle ledit au moins un résidu d'acide aminé supplémentaire ou inséré est choisi dans le groupe constitué de : G, N, I, S, T, C, Q, V, F, A, L, M, P, W et Y.

2. Enzyme subtilase isolée selon la revendication 1, dans laquelle ledit au moins un résidu d'acide aminé supplémentaire ou inséré comprend plus d'un résidu d'acide aminé supplémentaires ou insérés dans la boucle (b) du site actif.

3. Enzyme subtilase isolée selon la revendication 1 ou 2, dans laquelle ladite(lesdites) insertion(s) entre les positions 98 et 99 est (sont) combinée (s) avec une ou plusieurs modification(s) supplémentaire(s) au niveau de toute(s) autre (s) position(s).

4. Enzyme subtilase isolée selon la revendication 3, dans laquelle ladite(lesdites) modification(s) supplémentaire(s) est(sont) au niveau d'une ou plusieurs des positions 27, 36, 57, 76, 87, 97, 101, 104, 120, 123, 167, 170, 206, 218, 222, 224, 235 et 274.

5. Enzyme subtilase isolée selon l'une quelconque des revendications précédentes, dans laquelle ladite(lesdites) modification(s) est(sont) combinée(s) à une(des) modification(s) au niveau d'une ou plusieurs des positions 129, 131, 133 et 194.

6. Enzyme subtilase isolée selon l'une quelconque des revendications précédentes, la subtilase appartenant au sous-groupe I-S1.

7. Enzyme subtilase isolée selon la revendication 6, qui est choisie dans le groupe constitué de BSS168, BASBPN, BSSDY et BLSCAR.

8. Enzyme subtilase isolée selon l'une quelconque des revendications 1 à 5, la subtilase appartenant au sous-groupe I-S2.

9. Enzyme subtilase isolée selon la revendication 8, qui est choisie dans le groupe constitué de BLS147, BLS309, BAALKP et BYSYAB.

10. Enzyme subtilase isolée selon la revendication 1, 8 ou 9, laquelle enzyme comprend une insertion choisie dans le groupe constitué de

    A98AA,
    A98AT,
    A98AG,
    A98AS,
    A98AV,
    A98AC,
    A98AN,
    A98AQ,
    A98AF,
    A98AI,
    A98AL,
    A98AM,
    A98AP,
    A98AW,
    A98AY,
    A98SD,

A98TP, et
A98TW.

11. Enzyme subtilase isolée selon l'une quelconque des revendications 8 à 10, dans laquelle ladite(lesdites) modification(s) supplémentaire(s) est(sont) choisie(s) dans le groupe constitué de K27R, *36D, S57P, N76D, S87N, G97N, S101G, V104A, V104N, V104Y, H120D, N123S, Y167X, R170X, Q206E, N218S, M222S, M222A, T224S, K235L et T274A.

12. Enzyme subtilase isolée selon l'une quelconque des revendications 8 à 10, dans laquelle ladite(lesdites) modification(s) supplémentaire(s) est(sont) choisie(s) dans le groupe constitué de S101G+V104N, S87N+S101G+V104N, K27R+V104Y+N123S+T274A, N76D+S103A+V104I ou N76D+V104A, ou d'autres combinaisons de ces mutations (V104N, S101G, K27R, V104Y, N123S, T274A, N76D, V104A), en combinaison avec l'une quelconque ou plusieurs des substitutions et/ou insertions mentionnées dans l'une quelconque des revendications précédentes.

13. Enzyme subtilase isolée selon l'une quelconque des revendications 8 à 10, dans laquelle ladite(lesdites) modification(s) supplémentaire(s) est(sont) choisie(s) dans le groupe constitué en outre de P129K, P131H, A133P, A133D et A194P.

14. Enzyme subtilase isolée selon l'une quelconque des revendications précédentes, comprenant la modification choisie dans le groupe constitué de : A98AT+Y167A ; A98AT+S99SD ; A98ATD ; A98ADT A98AGSGG ; A98ATGSG ; A98AGGGS ; A98AGGGG ; A98ASGTG ; A98ATGTG ; S87G+A98AGGGS ; L96LD+A98AT ; G97D+A98AT ; G97E+A98AT ; A98AT+Y167A ; A98AT+R247K ; A98GP+S99A ; A98AS+A133E+T143K ; A98AT+A108C+A138C ; A98AT+Y167A+R170S+A194P ; A98GI+S99H+G100S+S101A.

15. Enzyme subtilase isolée selon la revendication 1, laquelle enzyme appartient ou sous-groupe I-S1 ayant la séquence en acides aminés :

```
1                    10                   20                   30
A-Q-T-V-P-Y-G-I-P-L-I-K-A-D-K-V-Q-A-Q-G-F-K-G-A-N-V-K-V-A-V
                     40                   50                   60
L-D-T-G-I-Q-A-S-H-P-D-L-N-V-V-G-G-A-S-F-V-A-G-E-A-*-Y-N-T-D
                     70                   80                   90
G-N-G-H-G-T-H-V-A-G-T-V-A-A-L-D-N-T-T-G-V-L-G-V-A-P-S-V-S-L
                   98a                    110                  120
Y-A-V-K-V-L-N-S-X-S-G-S-G-T-Y-S-G-I-V-S-G-I-E-W-A-T-T-N-G-M-D
                     130                  140                  150
V-I-N-M-S-L-G-G-P-S-G-S-T-A-M-K-Q-A-V-D-N-A-Y-A-R-G-V-V-V-V
                     160                  170                  180
A-A-A-G-N-S-G-S-S-G-N-T-N-T-I-G-Y-P-A-K-Y-D-S-V-I-A-V-G-A-V
                     190                  200                  210
D-S-N-S-N-R-A-S-F-S-S-V-G-A-E-L-E-V-M-A-P-G-A-G-V-Y-S-T-Y-P
                     220                  230                  240
T-S-T-Y-A-T-L-N-G-T-S-M-A-S-P-H-V-A-G-A-A-A-L-I-L-S-K-H-P-N
                     250                  260                  270
L-S-A-S-Q-V-R-N-R-L-S-S-T-A-T-Y-L-G-S-S-F-Y-Y-G-K-G-L-I-N-V
```

```
        275
   E-A-A-A-Q
```

**16.** Enzyme subtilase isolée selon la revendication 1, laquelle enzyme appartient ou sous-groupe I-S2 ayant la séquence en acides aminés :

```
1                   10                  20                  30
A-Q-S-V-P-W-G-I-S-R-V-Q-A-P-A-A-H-N-R-G-L-T-G-S-G-V-K-V-A-V-
                    40                  50                  60
L-D-T-G-I-*-S-T-H-P-D-L-N-I-R-G-G-A-S-F-V-P-G-E-P-*-S-T-Q-D
                    70                  80                  90
G-N-G-H-G-T-H-V-A-G-T-I-A-A-L-N-N-S-I-G-V-L-G-V-A-P-S-A-E-L-
                    98a                 110                 120
Y-A-V-K-V-L-G-A-X-S-G-S-G-S-V-S-S-I-A-Q-G-L-E-W-A-G-N-N-G-M-H-
                    130                 140                 150
V-A-N-L-S-L-G-S-P-S-P-S-A-T-L-E-Q-A-V-N-S-A-T-S-R-G-V-L-V-V-
                    160                 170                 180
A-A-S-G-N-S-G-A-*-G-S-I-S-*-*-*-Y-P-A-R-Y-A-N-A-M-A-V-G-A-T-
                    190                 200                 210
D-Q-N-N-N-R-A-S-F-S-Q-Y-G-A-G-L-D-I-V-A-P-G-V-N-V-Q-S-T-Y-P-
                    220                 230                 240
G-S-T-Y-A-S-L-N-G-T-S-M-A-T-P-H-V-A-G-A-A-A-L-V-K-Q-K-N-P-S-
                    250                 260                 270
W-S-N-V-Q-I-R-N-H-L-K-N-T-A-T-S-L-G-S-T-N-L-Y-G-S-G-L-V-N-A-
        275
E-A-A-T-R
```

**17.** Enzyme subtilase isolée selon la revendication 15 ou 16, dans laquelle X en position 98a est choisi dans le groupe constitué de T, A, G, S et P.

**18.** Séquence d'ADN isolée codant une enzyme subtilase selon l'une quelconque des revendications précédentes.

**19.** Vecteur d'expression comprenant une séquence d'ADN isolée selon la revendication 18.

**20.** Cellule hôte microbienne transformée avec un vecteur d'expression selon la revendication 19.

**21.** Hôte microbien selon la revendication 20, qui est une bactérie, de préférence un bacille, notamment *B. lentus.*

**22.** Hôte microbien selon la revendication 20, qui est un champignon ou une levure, de préférence un champignon filamenteux, notamment un *Aspergillus.*

**23.** Méthode pour produire une enzyme subtilase isolée selon l'une quelconque des revendications 1 à 17, dans laquelle un hôte selon l'une quelconque des revendications 20 à 22 est cultivé dans des conditions favorables à l'expression et la sécrétion de ladite enzyme subtilase, et l'enzyme subtilase est récupérée.

**24.** Composition comprenant une enzyme subtilase isolée selon l'une quelconque des revendications 1 à 17.

**25.** Composition selon la revendication 24, qui comprend en outre une cellulase, lipase, cutinase, oxydoréductase, une autre protéase ou une amylase.

**26.** Composition selon la revendication 24 ou 25, la composition étant une composition détergente.

**27.** Utilisation d'une enzyme subtilase isolée selon l'une quelconque des revendications 1 à 17 ou d'une composition d'enzyme selon la revendication 24 ou 25 dans un détergent pour linge et/ou pour vaisselle.

```
No: 1        10        20        30        40        50
a)  AQSVPYGVSQIKAPALHSQGYTGSNVKVAVIDSGIDSSHPDLKVAGGASM
b)  AQSVPWGISRVQAPAAHNRGLTGSGVKVAVLDTGI*STHPDLNIRGGASF


No:         60        70        80        90        100
a)  VPSETNPFQDNNSHGTHVAGTVAALNNSIGVLGVAPSASLYAVKVLGADG
b)  VPGEPST*QDGNGHGTHVAGTIAALNNSIGVLGVAPSAELYAVKVLGASG


No:         110       120       130       140       150
a)  SGQYSWIINGIEWAIANNMDVINMSLGGPSGSAALKAAVDKAVASGVVVV
b)  SGSVSSIAQGLEWAGNNGMHVANLSLGSPSPSATLEQAVNSATSRGVLVV


No:         160       170       180       190       200
a)  AAAGNEGTSGSSSTVGYPGKYPSVIAVGAVDSSNQRASFSSVGPELDVMA
b)  AASGNSG*AGS***ISYPARYANAMAVGATDQNNNRASFSQYGAGLDIVA


No:         210       220       230       240       250
a)  PGVSIQSTLPGNKYGAYNGTSMASPHVAGAAALILSKHPNWTNTQVRSSL
b)  PGVNVQSTYPGSTYASLNGTSMATPHVAGAAALVKQKNPSWSNVQIRNHL


No:         260       270  275
a)  ENTTTKLGDSFYYGKGLINVQAAAQ
b)  KNTATSLGSTNLYGSGLVNAEAATR
```

## Fig.1

No: 1                    10                   20                   30
a)  A-Q-S-V-P-Y-G-V-S-Q-I-K-A-P-A-L-H-S-Q-G-Y-T-G-S-N-V-K-V-A-V-

b)  A-Q-S-V-P-W-G-I-S-R-V-Q-A-P-A-A-H-N-R-G-L-T-G-S-G-V-K-V-A-V-


No:                      40                   50                   60
a)  I-D-S-G-I-D-S-S-H-P-D-L-K-V-A-G-G-A-S-M-V-P-S-E-T-N-P-F-Q-D-

b)  L-D-T-G-I-*-S-T-H-P-D-L-N-I-R-G-G-A-S-F-V-P-G-E-P-*-S-T-Q-D-


No:                      70                   80                   90
a)  N-N-S-H-G-T-H-V-A-G-T-V-A-A-L-N-N-S-I-G-V-L-G-V-A-P-S-A-S-L-

b)  G-N-G-H-G-T-H-V-A-G-T-I-A-A-L-N-N-S-I-G-V-L-G-V-A-P-S-A-E-L-

No:                      100                  110                  120
a)  Y-A-V-K-V-L-G-A-D-G-S-G-Q-Y-S-W-I-I-N-G-I-E-W-A-I-A-N-N-M-D-

b)  Y-A-V-K-V-L-G-A-S-G-S-G-S-V-S-S-I-A-Q-G-L-E-W-A-G-N-N-G-M-H-

No:                      130                  140                  150
a)  V-I-N-M-S-L-G-G-P-S-G-S-A-A-L-K-A-A-V-D-K-A-V-A-S-G-V-V-V-V-

b)  V-A-N-L-S-L-G-S-P-S-P-S-A-T-L-E-Q-A-V-N-S-A-T-S-R-G-V-L-V-V-

No:                      160                  170                  180
a)  A-A-A-G-N-E-G-T-S-G-S-S-S-T-V-G-Y-P-G-K-Y-P-S-V-I-A-V-G-A-V-

b)  A-A-S-G-N-S-G-A-*-G-S-I-S-*-*-*-Y-P-A-R-Y-A-N-A-M-A-V-G-A-T-

No:                      190                  200                  210
a)  D-S-S-N-Q-R-A-S-F-S-S-V-G-P-E-L-D-V-M-A-P-G-V-S-I-Q-S-T-L-P-

b)  D-Q-N-N-N-R-A-S-F-S-Q-Y-G-A-G-L-D-I-V-A-P-G-V-N-V-Q-S-T-Y-P-

No:                      220                  230                  240
a)  G-N-K-Y-G-A-Y-N-G-T-S-M-A-S-P-H-V-A-G-A-A-A-L-I-L-S-K-H-P-N-

b)  G-S-T-Y-A-S-L-N-G-T-S-M-A-T-P-H-V-A-G-A-A-A-L-V-K-Q-K-N-P-S-

No:                      250                  260                  270
a)  W-T-N-T-Q-V-R-S-S-L-E-N-T-T-T-K-L-G-D-S-F-Y-Y-G-K-G-L-I-N-V-

b)  W-S-N-V-Q-I-R-N-H-L-K-N-T-A-T-S-L-G-S-T-N-L-Y-G-S-G-L-V-N-A-

No:             275
a)  Q-A-A-A-Q

b)  E-A-A-T-R


# Fig.1a

```
No.:           10        20        30        40        50
a)   AQSVPYGVSQIKAPALHSQGYTGSNVKVAVIDSGIDSSHPDLKVAGGASM
g)   AQTVPYGIPLIKADKVQAQGFKGANVKVAVLDTGIQASHPDLNVVGGASF


No.:           60        70        80        90       100
a)   VPSETNPFQDNNSHGTHVAGTVAALNNSIGVLGVAPSASLYAVKVLGADG
g)   VAGEAYN*TDGNGHGTHVAGTVAALDNTTGVLGVAPSVSLYAVKVLNSSG


No.:          110       120       130       140       150
a)   SGQYSWIINGIEWAIANNMDVINMSLGGPSGSAALKAAVDKAVASGVVVV
b)   SGTYSGIVSGIEWATTNGMDVINMSLGGPSGSTAMKQAVDNAYARGVVVV


No.:          160       170       180       190       200
a)   AAAGNEGTSGSSSTVGYPGKYPSVIAVGAVDSSNQRASFSSVGPELDVMA
b)   AAAGNSGSSGNTNTIGYPAKYDSVIAVGAVDSNSNRASFSSVGAELEVMA


No.:          210       220       230       240       250
a)   PGVSIQSTLPGNKYGAYNGTSMASPHVAGAAALILSKHPNWTNTQVRSSL
b)   PGAGVYSTYPTSTYATLNGTSMASPHVAGAAALILSKHPNLSASQVRNRL


No.:          260       270  275
a)   ENTTTKLGDSFYYGKGLINVQAAAQ
b)   SSTATYLGSSFYYGKGLINVEAAAQ
```

## Fig. 2

```
No: 1                 10                  20                  30
a)  A-Q-S-V-P-Y-G-V-S-Q-I-K-A-P-A-L-H-S-Q-G-Y-T-G-S-N-V-K-V-A-V-

g)  A-Q-T-V-P-Y-G-I-P-L-I-K-A-D-K-V-Q-A-Q-G-F-K-G-A-N-V-K-V-A-V-


No:                   40                  50                  60
a)  I-D-S-G-I-D-S-S-H-P-D-L-K-V-A-G-G-A-S-M-V-P-S-E-T-N-P-F-Q-D-

g)  L-D-T-G-I-Q-A-S-H-P-D-L-N-V-V-G-G-A-S-F-V-A-G-E-A-*-Y-N-T-D-


No:                   70                  80                  90
a)  H-G-T-H-V-N-N-S-A-G-T-V-A-A-L-N-N-S-I-G-V-L-G-V-A-P-S-A-S-L-

g)  G-N-G-H-G-T-H-V-A-G-T-V-A-A-L-D-N-T-T-G-V-L-G-V-A-P-S-V-S-L-


No:                   100                 110                 120
a)  Y-A-V-K-V-L-G-A-D-G-S-G-Q-Y-S-W-I-I-N-G-I-E-W-A-I-A-N-N-M-D-

g)  Y-A-V-K-V-L-N-S-S-G-S-G-T-Y-S-G-I-V-S-G-I-E-W-A-T-T-N-G-M-D-


No:                   130                 140                 150
a)  V-I-N-M-S-L-G-G-P-S-G-S-A-A-L-K-A-A-V-D-K-A-V-A-S-G-V-V-V-V-

g)  V-I-N-M-S-L-G-G-P-S-G-S-T-A-M-K-Q-A-V-D-N-A-Y-A-R-G-V-V-V-V-

No:                   160                 170                 180
a)  A-A-A-G-N-E-G-T-S-G-S-S-S-T-V-G-Y-P-G-K-Y-P-S-V-I-A-V-G-A-V-

g)  A-A-A-G-N-S-G-S-S-S-G-N-T-N-T-I-G-Y-P-A-K-Y-D-S-V-I-A-V-G-A-V-

No:                   190                 200                 210
a)  D-S-S-N-Q-R-A-S-F-S-S-V-G-P-E-L-D-V-M-A-P-G-V-S-I-Q-S-T-L-P-

g)  D-S-N-S-N-R-A-S-F-S-S-V-G-A-E-L-E-V-M-A-P-G-A-G-V-Y-S-T-Y-P-

No:                   220                 230                 240
a)  G-N-K-Y-G-A-Y-N-G-T-S-M-A-S-P-H-V-A-G-A-A-A-L-I-L-S-K-H-P-N-

g)  T-S-T-Y-A-T-L-N-G-T-S-M-A-S-P-H-V-A-G-A-A-A-L-I-L-S-K-H-P-N-

No:                   250                 260                 270
a)  W-T-N-T-Q-V-R-S-S-L-E-N-T-T-T-K-L-G-D-S-F-Y-Y-G-K-G-L-I-N-V-

g)  L-S-A-S-Q-V-R-N-R-L-S-S-T-A-T-Y-L-G-S-S-F-Y-Y-G-K-G-L-I-N-V-

No:        275
a)  Q-A-A-A-Q

g)  E-A-A-A-Q
```

## Fig. 2a

95-103: VLGASGSGS

Fig. 3